(19) Europäisches Patentamt
European Patent Office
Office européen des brevets

(11) EP 0 956 278 B1

(12) **FASCICULE DE BREVET EUROPEEN**

(45) Date de publication et mention de la délivrance du brevet: **02.04.2003 Bulletin 2003/14**

(21) Numéro de dépôt: **98902027.6**

(22) Date de dépôt: **06.01.1998**

(51) Int Cl.[7]: **C07B 41/02**, C07C 29/143, C07C 35/08

(86) Numéro de dépôt international: **PCT/FR98/00010**

(87) Numéro de publication internationale: **WO 98/030517 (16.07.1998 Gazette 1998/28)**

(54) **PROCEDE DE REDUCTION D'UN COMPOSE CARBONYLE**

VERFAHREN ZUR REDUKTION EINER CARBONYLVERBINDUNG

METHOD FOR REDUCING A CARBONYL-CONTAINING COMPOUND

(84) Etats contractants désignés: **CH DE GB IT LI NL**

(30) Priorité: **06.01.1997 FR 9700042**

(43) Date de publication de la demande: **17.11.1999 Bulletin 1999/46**

(73) Titulaire: **RHODIA CHIMIE**
**92408 Courbevoie Cédex (FR)**

(72) Inventeurs:
- **JACQUOT, Roland**
  **F-69110 Sainte Foy lès Lyon (FR)**
- **SPAGNOL, Michel**
  **F-69008 Lyon (FR)**

(74) Mandataire: **Dutruc-Rosset, Marie-Claude**
**Rhodia Services,**
**Direction de la Propriété Industrielle,**
**40, rue de la Haie-Coq**
**93306 Aubervilliers Cedex (FR)**

(56) Documents cités:
- **E.J. CREYGHTON, ET AL.: "Stereoselective reduction of 4-tert-butylcyclohexanone to cis-4-tert-butylcyclohexanol catalysed by zeolite BEA" JOURNAL OF THE CHEMICAL SOCIETY, CHEMICAL COMMUNICATIONS, no. 18, 21 septembre 1995, LETCHWORTH, GB, pages 1859-1860, XP002038921 cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 956 278 B1

## Description

La présente invention a pour objet un procédé de réduction d'un composé carbonylé. Plus précisément, l'invention concerne un procédé de réduction d'un aldéhyde et/ou d'une cétone.

Il existe différents procédés de réduction de composés carbonylés en alcools. En particulier, il est connu de réduire les aldéhydes et les cétones en alcools, selon un procédé d'hydrogénation, en présence de nickel Raney.

Le problème qui se pose est que la réduction d'un aldéhyde ou d'une cétone conduite selon le procédé précité, est une réaction non stéréosélective et l'on obtient alors un mélange de stéréoisomères. Il en est ainsi, lorsque l'on conduit la réduction d'une cyclohexanone substituée, conduisant ainsi à un mélange thermodynamique des stéréoisomères.

L'objectif de la présente invention est de fournir un procédé simple de réduction applicable à tout composé carbonylé, et plus particulièrement aux composés carbonylés, pour lesquels l'obtention d'un stéréoisomère majoritaire est requise.

Par ailleurs, Van Bekkum et al ont décrit la réduction du 4-tert-butylcyclohexanone en cis-4-tert-butylcyclohexanol catalysée par une zéolithe BEA [J. Chem. Soc., Chem. Commun, p.1859-60 (1995)].

La mise en oeuvre dudit catalyseur à l'échelle industrielle pose un problème car la productivité du catalyseur est insuffisante si bien qu'il serait nécessaire de prévoir une taille de réacteur très conséquente.

L'objet de la présente invention est de fournir un procédé permettant d'obvier aux inconvénients précités.

Il a maintenant été trouvé et c'est ce qui constitue l'objet de la présente invention, un procédé de réduction d'un composé carbonylé en alcool correspondant, par réaction dudit composé carbonylé avec un alcool en présence d'un catalyseur zéolithique caractérisé par le fait qu'il consiste :

- à effectuer le mélange d'une manière quelconque du composé carbonylé et de l'alcool.
- à faire passer ledit mélange sur un lit catalytique comprenant au moins une zéolithe,
- à soumettre le mélange réactionnel issu du lit catalytique à une recirculation sur lit catalytique, en un nombre de fois suffisant pour obtenir le taux de conversion du substrat souhaité.

Dans le procédé de l'invention, on met donc en oeuvre un composé carbonylé, aldéhyde ou cétone et un alcool.

Plus précisément, la présente invention a pour objet un procédé de réduction d'un composé carbonylé répondant plus particulièrement à la formule générale (I) :

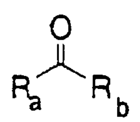 (I)

dans ladite formule (I) :

- $R_a$ et $R_b$, identiques ou différents, représentent un atome d'hydrogène ou un radical hydrocarboné monovalent, éventuellement substitué ayant de 1 à 40 atomes de carbone ; au plus l'un des radicaux $R_a$ ou $R_b$ est un atome d'hydrogène,
- $R_a$ et $R_b$ peuvent former un cycle comprenant éventuellement un autre hétéroatome.

Plus précisément, $R_a$ et $R_b$, peuvent représenter un radical hydrocarboné monovalent, substitué ou non qui peut être un radical aliphatique acyclique saturé ou insaturé, linéaire ou ramifié ; un radical carbocyclique ou hétérocyclique saturé, insaturé ou aromatique, monocyclique ou polycyclique.

$R_a$ et $R_b$ peuvent prendre diverses significations. Différents exemples sont donnés ci-après mais ils ne sont en aucun cas limitatif.

Dans les composés de formule (I), $R_a$ et $R_b$ représentent préférentiellement un radical aliphatique acyclique, saturé ou insaturé, linéaire ou ramifié ayant de préférence de 1 à 12 atomes de carbone, saturé ou comprenant une à plusieurs insaturations sur la chaîne, généralement, 1 à 3 insaturations qui peuvent être des doubles liaisons simples ou conjuguées ou des triples liaisons.

$R_a$ et $R_b$ représentent plus particulièrement un radical alkyle, alcényle, alcadiényle, linéaire ou ramifié ayant de préférence de 1 à 12 atomes de carbone.

La chaîne hydrocarbonée peut être éventuellement :

- interrompue par l'un des groupes suivants Z :

  -O- ; -CO- ; COO- ; $-NR_1-$ ; $-CO-NR_1-$ ; -S- ; $-SO_2-$ ; $-NR_1-CO-$ ; dans lesdites formules $R_1$ représente un atome d'hydrogène, un groupe alkyle linéaire ou ramifié ayant de 1 à 6 atomes de carbone, de préférence, un radical méthyle ou éthyle,
- et/ou porteuse de l'un des substituants suivants :

  - OH ; -COOH ; -COOX ; $-CO-N(R_1)(R_2)$ ; $-COOR_1$ ; -CHO ; $-COR_1$ ; $-NO_2$ ; -X ; $-CF_3$.

  dans ces formules, X représente un atome d'halogène, de préférence, un atome de chlore ou de brome et $R_2$ a la même signification que $R_1$ donnée précédemment.

Il est également possible que $R_a$ et $R_b$ représentent un radical aliphatique acyclique, saturé ou insaturé, linéaire ou ramifié éventuellement porteur d'un substituant cyclique. Par cycle, on entend un cycle carbocyclique ou hétérocyclique, saturé, insaturé ou aromatique.

Le radical aliphatique acyclique peut être relié au cycle par un lien valentiel ou par l'un des groupes Z précités.

Comme exemples de substituants cycliques, on peut envisager des substituants cycloaliphatiques, aromatiques ou hétérocycliques, notamment cycloaliphatiques comprenant 6 atomes de carbone dans le cycle ou benzéniques, ces substituants cycliques étant eux-mêmes éventuellement porteurs d'un ou plusieurs substituants.

Comme exemples de tels radicaux, on peut mentionner, entre autres, le radical benzyle.

Dans la formule générale (I), $R_a$ et $R_b$ peuvent représenter un radical carbocyclique, monocyclique. Le nombre d'atomes de carbone dans le cycle peut varier largement de 3 à 8 atomes de carbone mais il est de préférence égal à 5 ou 6 atomes de carbone.

Le carbocycle peut être saturé ou comprenant 1 ou 2 insaturations dans le cycle, de préférence de 1 à 2 doubles liaisons.

Comme exemples préférés de radicaux $R_a$ et $R_b$, on peut citer les radicaux cyclohexyle ou cyclohexène-yle.

Dans le cas où $R_a$ et $R_b$ représente un radical carbocyclique, monocyclique, saturé ou insaturé, il est possible que l'un ou plusieurs des atomes du carbone du cycle soient remplacés par un hétéroatome, de préférence, oxygène, azote ou soufre ou par un groupe fonctionnel, de préférence carbonyle ou ester, conduisant ainsi à un composé hétérocyclique, monocyclique. Le nombre d'atomes dans le cycle peut varier largement de 3 à 8 atomes mais il est de préférence égal à 5 ou 6 atomes.

Les radicaux $R_a$ et $R_b$ peuvent être également carbocyclique, polycyclique, de préférence bicyclique ce qui signifie qu'au moins deux cycles ont deux atomes de carbone en commun. Dans le cas des radicaux polycycliques, le nombre d'atomes de carbone dans chaque cycle varie entre 3 et 6 : le nombre total d'atomes de carbone étant égal de préférence à 7.

On donne ci-après des exemples de structure bicyclique, couramment rencontrée :

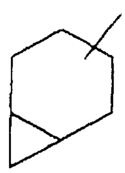
[4,1,0]

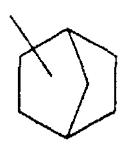
[2,2,1]

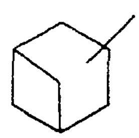
[3,1,1]

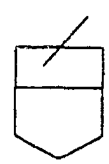
[3,2,0]

Les radicaux $R_a$ et $R_b$ peuvent être également hétérocyclique, polycyclique, de préférence bicyclique ce qui signifie qu'au moins deux cycles ont deux atomes en commun. Dans ce cas, le nombre d'atomes dans chaque cycle varie entre 3 et 6 et est plus préférentiellement égal à 5 ou 6.

Les radicaux $R_a$ et $R_b$ peuvent représenter préférentiellement un radical carbocyclique aromatique, et notamment benzénique répondant à la formule générale (II) :

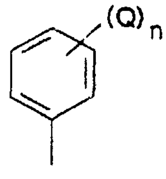
(II)

dans ladite formule (II) :

- n est un nombre entier de 0 à 5, de préférence de 0 à 3,
- Q représente $R_3$, l'un des groupes ou fonctions suivantes :

  . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  . un radical alcényle linéaire ou ramifié ayant de 2 à 6 atomes de carbone, de préférence, de 2 à 4 atomes de carbone, tel que vinyle, allyle,
  . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  . un groupe acyle ayant de 2 à 6 atomes de carbone,
  . un radical de formule :

$$-R_5-OH$$

$$-R_5-COOR_7$$

$$-R_5-CHO$$

$$-R_5-NO_2$$

$$-R_5-CN$$

$$-R_5-N(R_7)(R_8)$$

$$-R_5-CO-N(R_7)(R_8)$$

$$-R_5-SH$$

$$-R_5-X$$

$$-R_5-CF_3$$

  dans lesdites formules, $R_5$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; les radicaux $R_7$ et $R_8$, identiques ou différents, représentent un atome d'hydrogène ou un radical alkyle linéaire ou ramifié ayant de 1 à 6 atomes de carbone ; X symbolise un atome d'halogène, de préférence un atome de chlore, de brome ou de fluor.

- Q représente $R_4$, l'un des radicaux plus complexes suivants :

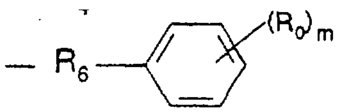

dans lequel:

- m est un nombre entier de 0 à 5, de préférence de 0 à 3,

  . $R_0$ ayant la signification donnée précédemment pour $R_3$,
  . $R_6$ représente un lien valentiel ; un groupe hydrocarboné divalent, linéaire ou ramifié, saturé ou insaturé ayant de 1 à 6 atomes de carbone tel que par exemple, méthylène, éthylène, propylène, isopropylène, isopropylidène ou l'un des groupes suivants dénommés Z :
  -O- ; -CO- ; COO- ; $-NR_7-$ ; $-CO-NR_7-$ ; -S- ; $-SO_2-$ ; $-NR_7-CO-$; dans lesdites formules $R_7$ représente un atome d'hydrogène, un groupe alkyle linéaire ou ramifié ayant de 1 à 6 atomes de carbone, de préférence, un radical méthyle ou éthyle.

Comme exemples de radicaux $R_a$ et $R_b$ répondant à la formule (II), on peut mentionner plus précisément les radicaux phényle, tolyle ou xylyle, 1-méthoxyphényle, 2-nitrophényle et les radicaux biphényle, 1,1'-méthylènebiphényle, 1,1'-isopropylidènebiphényle, 1,1'-carboxybiphényle, 1,1'-oxybiphényle, 1,1'-iminobiphényle : lesdits radicaux pouvant être substitués par un ou plusieurs radicaux Q tels que prédéfinis.

$R_a$ et $R_b$ peuvent également représenter un radical hydrocarboné aromatique polycyclique ; les cycles pouvant former entre eux des systèmes ortho-condensés, ortho- et péri-condensés. On peut citer plus particulièrement, un radical naphtalénique.

Dans la formule générale (I), $R_a$ et $R_b$ peuvent également représenter un radical hétérocyclique aromatique, comportant notamment 5 ou 6 atomes dans le cycle dont 1 ou 2 hétéroatomes tels que les atomes d'azote, de soufre et d'oxygène.

$R_a$ et $R_b$ peuvent aussi représenter un radical hétérocyclique aromatique polycyclique défini comme étant soit un radical constitué par au moins 2 hétérocycles aromatiques ou non contenant au moins un hétéroatome dans chaque cycle et formant entre eux des systèmes ortho- ou ortho- et péricondensés ou soit un radical constitué par au moins un cycle hydrocarboné aromatique ou non et au moins un hétérocycle aromatique ou non formant entre eux des systèmes ortho- ou ortho- et péricondensés.

Il est à noter que si le radical $R_a$ et $R_b$ comprend un cycle quelconque, il est possible que ce cycle porte un substituant. La nature du substituant est quelconque dans la mesure où il n'interfère pas au niveau du produit désiré. Les substituants portés le plus souvent par le cycle sont un ou plusieurs radicaux alkyle ou alkoxy ayant de préférence de 1 à 4 atomes de carbone, de préférence trois radicaux méthyle, un radical méthylène (correspondant à une liaison exocyclique), un radical alcényle, de préférence un radical isopropène-yle, un atome d'halogène, de préférence chlore ou brome.

Parmi tous les radicaux $R_a$ et $R_b$ précités, $R_a$ représente un radical phényle éventuellement porteur d'un groupe alkyle ou alkoxy ayant de 1 à 4 atomes de carbone, un groupe trihalogénométhyle ou un atome d'halogène et $R_b$, un atome d'hydrogène.

Dans la formule (I), il est possible que $R_a$ et $R_b$ forment ensemble et avec l'atome de carbone auquel ils ont attachés, un système cyclique, monocyclique ou polycyclique.

Ainsi, le composé de formule (I) peut être une cétone cyclique.

Le nombre de groupe carbonyle présent dans le substrat de départ peut donc être supérieur à 1, par exemple égal à 2 et l'on est alors en présence d'une dione.

Etant donné que la cétone cyclique de départ mise en oeuvre dans le procédé de l'invention peut être polycyclique, notamment bicyclique, il s'en suit que le nombre de groupes carbonyle dans le composé cétonique de départ peut être égal à 3, voire même à 4 ou plus.

La cétone cyclique mise en oeuvre dans le procédé de l'invention peut donc être une cétone mono- ou polycarbonylée. Il peut s'agir d'une cétone monocyclique ou polycyclique.

Le procédé de l'invention convient tout à fait bien aux cétones cycliques répondant à la formule générale (Ia) :

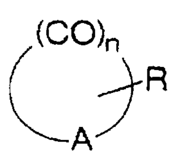

(Ia)

dans laquelle :

- A symbolise le reste d'un cycle formant tout ou partie d'un système monocyclique ou polycyclique comprenant au

moins un groupe carbonyle,

- R représente un atome d'hydrogène ou un ou plusieurs substituants, identiques ou différents,
- n est un nombre de préférence égal à 1 ou 2.

On précisera, sans pour autant limiter la portée de l'invention, que le reste A éventuellement substitué représente préférentiellement, le reste :

- d'un composé monocyclique, carbocyclique, saturé ou insaturé,
- d'un composé polycyclique comprenant au moins deux carbocycles, saturés et/ou insaturés,
- d'un composé polycyclique comprenant au moins deux cycles saturés et/ou insaturés : un ou plusieurs des atomes de carbone pouvant être remplacés par un hétéroatome,
- d'un composé polycyclique comprenant au moins deux carbocycles dont l'un d'eux est aromatique.

La cétone cyclique de formule (Ia) peut donc être un composé monocyclique ou polycyclique.
Lorsqu'il s'agit d'un composé monocyclique, le nombre d'atomes de carbone dans le cycle peut varier largement de 3 à 20 atomes de carbone mais il est de préférence de 5 ou 6 atomes de carbone.
Le carbocycle peut être saturé ou comprenant 1 ou 2 insaturations dans le cycle, de préférence de 1 à 2 doubles liaisons qui sont le plus souvent en position $\alpha$ du groupe carbonyle.
Le composé peut être également polycyclique, de préférence bicyclique ce qui signifie qu'au moins deux cycles ont deux atomes de carbone en commun.
Dans le cas des composés polycycliques, la condensation en carbone de chaque cycle est plus faible, généralement de 3 à 8 mais est égale, de préférence à 5 ou 6 atomes de carbone.
Le composé polycyclique peut comprendre au moins deux cycles saturés et/ou insaturés : un ou plusieurs (de préférence deux) des atomes de carbone pouvant être remplacés par un hétéroatome, de préférence un atome d'oxygène ou d'azote.
Le composé polycyclique peut comprendre au moins deux carbocycles dont l'un d'eux est aromatique, le cycle aromatique étant de préférence un cycle benzénique.
La cétone cyclique de formule (Ia) peut être porteuse d'un ou plusieurs substituants.
Le nombre de substituants présents sur le cycle dépend de la condensation en carbone du cycle et de la présence ou non d'insaturations sur le cycle.
Le nombre maximum de substituants susceptibles d'être portés par un cycle, est aisément déterminé par l'Homme du Métier.
En ce qui concerne la nature des substituants, des exemples de substituants sont donnés ci-après mais cette liste ne présente pas de caractère limitatif.
N'importe quel substituant peut être présent sur le cycle dans la mesure où il n'interfère pas au niveau du produit désiré. On donne ci-après des exemples de substituants qui peuvent être portés par le reste A :

- R peut représenter $R_9$, l'un des groupes suivants :

  . un radical aliphatique acyclique, linéaire ou ramifié, ayant de 1 à 20 atomes de carbone, saturé ou comprenant une ou plusieurs insaturations sur la chaîne, de préférence 1 à 3 insaturations qui sont de préférence, des doubles liaisons simples ou conjuguées ; la chaîne hydrocarbonée pouvant être éventuellement :

  . interrompue par l'un des groupes suivants dénommés Z :
  $-O-$; $-CO-$; $COO-$; $-NR_{10}-$; $-CO-NR_{10}-$; $-S-$; $-SO_2-$ ;
  dans lesdites formules $R_{10}$ représente un atome d'hydrogène, un radical alkyle linéaire ou ramifié ayant de 1 à 6 atomes de carbone,
  . et/ou porteuse de l'un des substituants suivants :
  $-OH$ ; $-CN$ ; $-N[R_{10}]_2$; $-COOR_{10}$; $-CF_3$ ou $-X$ ;
  dans lesdites formules, les radicaux $R_{10}$, identiques ou différents, représentent un atome d'hydrogène, un radical alkyle linéaire ou ramifié ayant de 1 à 6 atomes de carbone et X représente. un atome d'halogène, de préférence, de fluor, de chlore, de brome,
  . un radical du type $=R_{11}$, dans lequel $R_{11}$ représente un radical alkylidène ayant de 1 à 6 atomes de carbone, un radical de formule $=C(CN)_2$ ou un radical cycloalkylidène ou cycloalcénylidène ayant 5 ou 6 atomes de carbone ou un radical benzylidène éventuellement substitué, de préférence par un atome d'halogène X,
  . un radical alkoxy linéaire ou ramifié ayant de 1 à 6 atomes de carbone,
  . deux atomes successifs du cycle peuvent être reliés entre eux par un pont époxy ou par un pont du type alkylène dioxy ayant de 1 à 4 atomes de carbone, de préférence les radicaux méthylène dioxy, éthylène dioxy,

propylène dioxy,

- . un groupe OH,
- . un groupe $COOR_{10}$, $R_{10}$ représentant un atome d'hydrogène ou un radical alkyle ayant de 1 à 6 atomes de carbone, de préférence méthyle, éthyle,
- . un groupe CN,
- . un atome d'halogène, de préférence, de fluor, de chlore, de brome.
- . un groupe $-CF_3$.

- R peut représenter $R_{12}$, l'un des groupes plus complexes suivants :

- . un radical carbocyclique saturé ou insaturé ayant de 4 à 7 atomes de carbone, de préférence un radical cyclopentyle, cyclohexyle, cyclopentène-2 yle, cyclopentène-3 yle, cyclohexène-1 yle, cyclohexène-2 yle, cyclohexène-3 yle,
- . un radical de formule

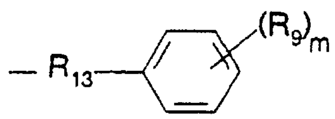

dans lequel $R_{13}$ représente un lien valentiel ou un radical hydrocarboné divalent, linéaire ou ramifié, saturé ou insaturé ayant de 1 à 6 atomes de carbone tel que par exemple, méthylène, éthylène, propylène, isopropylène, isopropylidène et $R_9$ ayant la signification donnée précédemment et m est un nombre entier de 0 à 4,

- . un radical $-R_{13}-Z-R_{14}$ dans lequel Z et $R_{13}$ ont la signification donnée précédemment, $R_{14}$ représente un radical alkyle linéaire ou ramifié ayant de 1 à 6 atomes de carbone ou un radical de formule

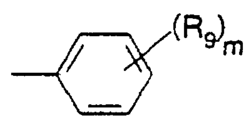

dans lequel $R_9$ et m ont la signification donnée précédemment,

- . un radical de type spiro de formule :

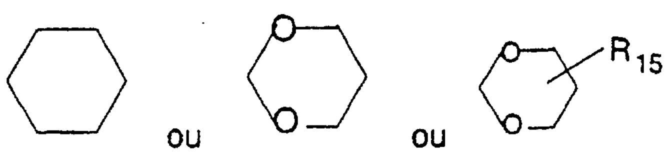

dans laquelle $R_{15}$ représente un ou plusieurs radicaux alkyle, linéaires ou ramifiés ayant de 1 à 6 atomes de carbone.

Plus précisément, dans la formule (Ia), les divers symboles précédents peuvent prendre les significations données ci-après.

A peut représenter le reste d'un composé carbocyclique monocyclique saturé, ayant de 3 à 20 atomes de carbone. Il peut y avoir présence d'un ou de deux groupes carbonyle sur le cycle. Le groupe carbonyle est de préférence porté par un carbocycle saturé ayant de 5 ou 6 atomes de carbone.

Le carbocycle saturé peut porter des substituants. Le nombre de substituants sur chaque cycle, peut varier largement de 1 à 5. Il est généralement de 1 ou 2.

Comme exemples spécifiques de substituants on peut mentionner :

- . un radical alkyle linéaire ou ramifié ayant de 1 à 15 atomes de carbone, de préférence un radical méthyle, éthyle, n-propyle, isopropyle, n-butyle, sec-butyle, tert-butyle, n-pentyle, isopentyle, tert-pentyle, n-hexyle, n-heptyle,

. un radical alkyle ayant de 1 à 15 atomes de carbone porteur d'un groupe fonctionnel, de préférence une fonction OH, CN, $N[R_{10}]_2$, $COOR_{10}$ avec $R_{10}$, identiques ou différents, représentant un atome d'hydrogène, un radical alkyle linéaire ou ramifié ayant de 1 à 6 atomes de carbone : la chaîne alkyle pouvant être interrompue par un atome d'oxygène ou un groupe carbonyle, carboxy ou amino éventuellement substitué et l'on peut citer plus particulièrement un radical de formule $-NHCH_3$ ou $-N(CH_3)_2$, un radical de formule $-CH_2-CH_2-CN$, un radical de formule $-CH_2-CO-(CH_2)_4-COOH$, un radical de formule $COCH(CH_3)_2$, un radical de formule $-(CH_2)_6-COOH$, un radical de formule $-CH_2-COOCH_3$, un radical de formule $-CH_2-COOC_2H_5$, un radical de formule $-CH_2-CH_2-COOCH_3$, un radical de formule $-(CH_2)_6-COOCH_3$, un radical de formule $-(CH_2)_6-COOC_2H_5$, un radical de formule $-(CH_2)_5-COOC_2H_5$, un radical de formule $-CO-CH_3$, un radical de formule $-C(CH_3)_2-CO-CH_3$, un radical de formule $-CH_2-CH_2-CO-(CH_2)_4-CH_3$,

. un radical alcénylène ou alkylidène, linéaire ou ramifié, comportant une ou deux doubles liaisons ayant de 1 à 15 atomes de carbone, de préférence, un radical de formule $-CH_2-CH=CH_2$, un radical de formule $-C(CH_3)=CH_2$, un radical de formule $-CH_2-CH=CH-C_2H_5$, un radical de formule $-CH_2-CH=CH-(CH_2)_2-CH_3$, un radical de formule $-CH=CH-(CH_2)_4-CH_3$, un radical de formule $-CH_2-CH=C(CH_3)_2$, un radical de formule $-CH_2-CH=CCH_3-(CH_2)_2-CH=C(CH_3)_2$,

. un radical $=C(CH_3)_2$ ou $=CH-(CH_2)_2-CH_3$,

. un radical alcénylène ou alkylidène, linéaire ou ramifié, comportant une ou deux doubles liaisons ayant de 1 à 15 atomes de carbone porteur d'un groupe fonctionnel, de préférence une fonction OH, CN, $NH_2$, $COOR_{10}$ dans laquelle $R_{10}$ a la signification donnée précédemment : la chaîne insaturée pouvant être interrompue par un atome d'oxygène, un groupe carbonyle ou carboxy et l'on peut mentionner plus spécialement un radical de formule $-CH_2-CH=CH-(CH_2)_3-COOH$, un radical de formule $-CH=CH-C(CH_3)=CH-COOH$, un radical de formule $-CH_2-CH=CH-(CH_2)_3-COOCH_3$, un radical de formule $-CH=CH-C(CH_3)=CH-COOCH_3$, un radical de formule $-CH=CH-CO-CH_3$, un radical de formule $-CH=CH-CO-(CH_2)_4-CH_3$ ou un radical de formule $-CH=CH-CHOH-(CH_2)_4-CH_3$,

. un radical alkoxy linéaire ou ramifié ayant de 1 à 6 atomes de carbone, de préférence un radical méthoxy, éthoxy,

. deux atomes successifs du cycle peuvent être reliés entre eux par un pont époxy ou par un pont tel que les radicaux méthylène dioxy, éthylène dioxy, propylène dioxy,

. un radical de type spiro de formule

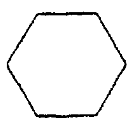 ou 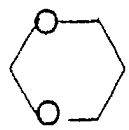 ou 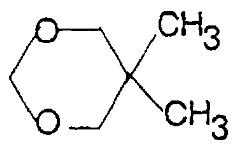

. un radical de formule

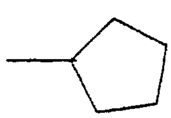 ou 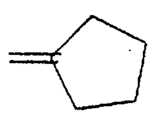 ou 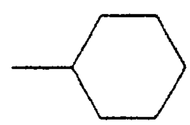 ou 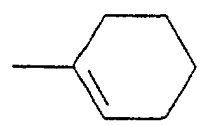

. un radical de formule

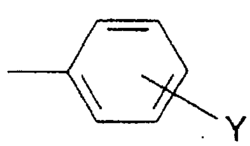 ou 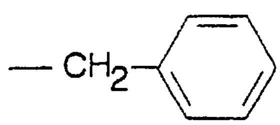 ou 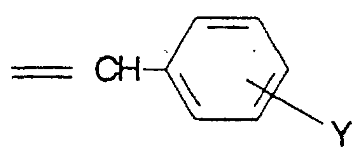

Y représentant un atome d'hydrogène, un atome d'halogène, de préférence de fluor ou de chlore,

. un radical de formule

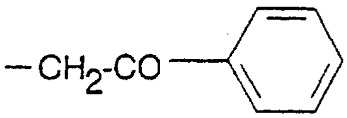

ou

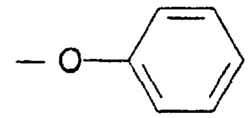

- un groupe OH,
- un groupe $COOR_{10}$, $R_{10}$ représentant un atome d'hydrogène ou un radical alkyle ayant de 1 à 6 atomes de carbone, de préférence méthyle, éthyle,
- un groupe CN,
- un atome d'halogène, de préférence, de fluor, de chlore, de brome.

A titre illustratif, on peut citer plus particulièrement les composés de formule suivante :

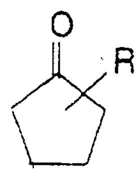
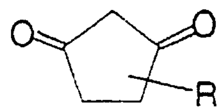
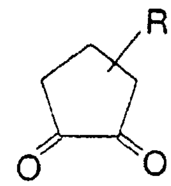
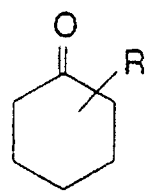
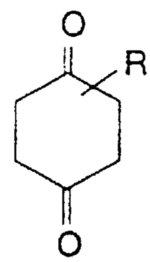
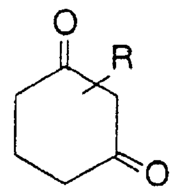
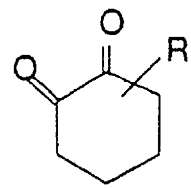

A peut représenter le reste d'un composé carbocyclique monocyclique insaturé, ayant de 4 à 20 atomes de carbone. Il peut y avoir présence d'un ou de deux groupes carbonyle sur le cycle. Le groupe carbonyle est de préférence porté par un carbocycle insaturé ayant de 5 ou 6 atomes de carbone.
Le carbocycle insaturé peut porter des substituants. Le nombre de substituants sur chaque cycle, peut varier largement de 1 à 5. Il est généralement: de 1 ou 2.
Comme exemples spécifiques de substituants on peut mentionner :

- un radical alkyle linéaire ou ramifié ayant de 1 à 15 atomes de carbone, de préférence un radical méthyle, éthyle, n-propyle, isopropyle, n-butyle, sec-butyle, tert-butyle, n-pentyle, isopentyle, tert-pentyle, n-hexyle, n-heptyle,
- un radical alkyle ayant de 1 à 15 atomes de carbone porteur d'un groupe fonctionnel, de préférence une fonction OH, CN, $N[R_{10}]_2$, $COOR_{10}$ avec $R_{10}$, identiques ou différents, représentant un atome d'hydrogène, un radical alkyle linéaire ou ramifié ayant de 1 à 6 atomes de carbone : la chaîne alkyle pouvant être interrompue par un atome d'oxygène ou un groupe carbonyle, carboxy ou amino éventuellement substitué et l'on peut citer plus particulièrement un radical de formule $-NHCH_3$ ou $-N(CH_3)_2$, un radical de formule $-CH_2-CH_2-CN$, un radical de formule $-CH_2-CO-(CH_2)_4-COOH$, un radical de formule $COCH(CH_3)_2$, un radical de formule $-(CH_2)_6-COOH$, un radical de formule $-CH_2-COOCH_3$, un radical de formule $-CH_2-COOC_2H_5$, un radical de formule $-CH_2-CH_2-COOCH_3$, un radical de formule $-(CH_2)_6-COOCH_3$, un radical de formule $-(CH_2)_6-COOC_2H_5$, un radical de formule $-(CH_2)_5-COOC_2H_5$, un radical de formule $-CO-CH_3$, un radical de formule $-C(CH_3)_2-CO-CH_3$, un radical de formule $-CH_2CH_2-CO-(CH_2)_4-CH_3$,
- un radical alcénylène ou alkylidène, linéaire ou ramifié, comportant une ou deux doubles liaisons ayant de 1 à 15 atomes de carbone, de préférence, un radical de formule $-CH_2-CH=CH_2$, un radical de formule $-C(CH_3)=CH_2$, un radical de formule $-CH_2-CH=CH-C_2H_5$, un radical de formule $-CH_2-CH=CH-(CH_2)_2-CH_3$, un radical de formule $-CH=CH-(CH_2)_4-CH_3$, un radical de formule $-CH_2-CH=C(CH_3)_2$, un radical de formule $-CH_2-CH=CCH_3-(CH_2)_2-CH=C(CH_3)_2$,
- un radical $=C(CH_3)_2$ ou $=CH-(CH_2)_2-CH_3$,
- un radical alcénylène ou alkylidène, linéaire ou ramifié, comportant une ou deux doubles liaisons ayant de 1 à 15 atomes de carbone porteur d'un groupe fonctionnel, de préférence une fonction OH, CN, $NH_2$, $COOR_{10}$ dans

laquelle $R_{10}$ a la signification donnée précédemment : la chaîne insaturée pouvant être interrompue par un atome d'oxygène, un groupe carbonyle ou carboxy et l'on peut mentionner plus spécialement un radical de formule $-CH_2-CH=CH-(CH_2)_3-COOH$, un radical de formule $-CH=CH-C(CH_3)=CH-COOH$, un radical de formule $-CH_2-CH=CH-(CH_2)_3-COOCH_3$, un radical de formule $-CH=CH-C(CH_3)=CH-COOCH_3$, un radical de formule $-CH=CH-CO-CH_3$, un radical de formule $-CH=CH-CO-(CH_2)_4-CH_3$ ou un radical de formule $-CH=CH-CHOH-(CH_2)_4-CH_3$,

. un radical alkoxy linéaire ou ramifié ayant de 1 à 6 atomes de carbone, de préférence un radical méthoxy, éthoxy,
. un radical de formule

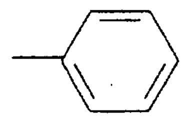

. un groupe OH,
. un groupe $COOR_{10}$, $R_{10}$ représentant un atome d'hydrogène ou un radical alkyle ayant de 1 à 6 atomes de carbone, de préférence méthyle, éthyle,
. un atome d'halogène, de préférence, de fluor, de chlore, de brome.

A titre illustratif, on peut citer plus particulièrement les composés de formule suivante :

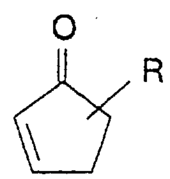 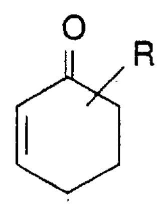 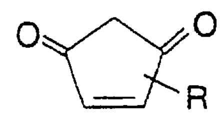 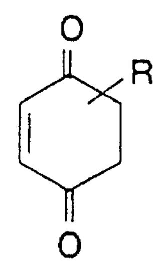

A peut représenter le reste d'un composé carbocyclique polycyclique, de préférence, bicyclique comprenant deux carbocycles saturés, ayant chacun de préférence de 4 à 8 atomes de carbone. Il peut y avoir présence d'un groupe carbonyle sur l'un ou les deux cycles. Il est également possible qu'il y ait deux groupes carbonyle sur le même cycle. Le groupe carbonyle est de préférence porté par un ou deux carbocycles saturés ayant de 5 ou 6 atomes de carbone.

Dans ces composés polycycliques, un ou plusieurs atomes de carbone, de préférence deux, peuvent être remplacés par un hétéroatome, de préférence un atome d'azote ou d'oxygène.

Le ou les cycles de ce composé polycyclique peuvent porter des substituants. Le nombre de substituants sur chaque cycle est généralement de 1 à 4, de préférence 1 ou 2. Comme exemples de substituants couramment rencontrés, on peut citer :

. un radical alkyle linéaire ou ramifié ayant de 1 à 6 atomes de carbone, de préférence un radical méthyle, isopropyle,
. un radical de formule $-CH_2Br$,
. un radical alkoxy linéaire ou ramifié ayant de 1 à 6 atomes de carbone, de préférence un radical méthoxy,
. un radical de formule $=CH_2$,
. un groupe OH,
. un groupe -COOH,
. un atome d'halogène, de préférence, de fluor, de chlore, de brome,
. un groupe $CF_3$

A titre illustratif, on peut citer plus particulièrement les composés de formule suivante :

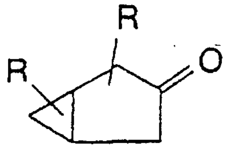
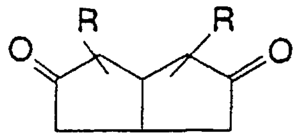
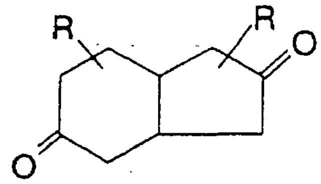

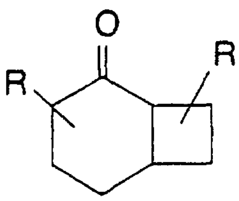
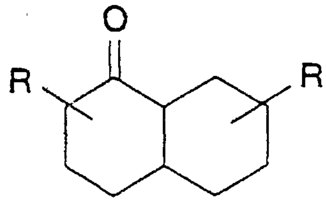
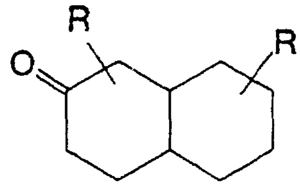

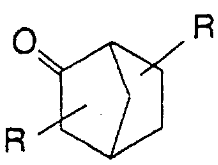
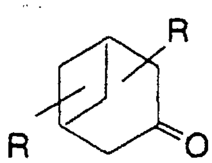
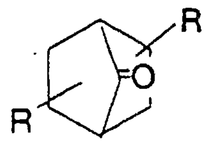
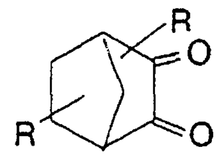

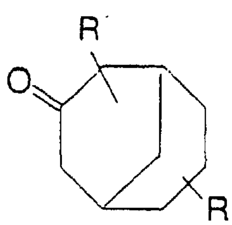
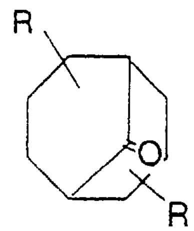
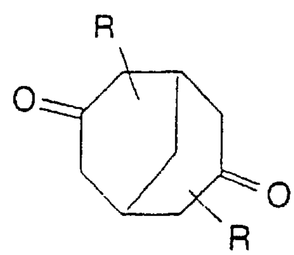

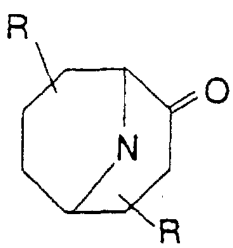
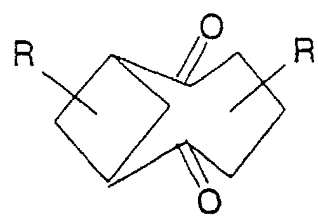

A peut représenter le reste d'un composé carbocyclique bicyclique comprenant deux carbocycles, ayant chacun de préférence de 4 à 7 atomes de carbone, l'un saturé, l'autre insaturé, généralement avec une seule double liaison. Le groupe carbonyle peut intervenir aussi bien dans le cycle saturé qu'insaturé ou sur les deux. Le groupe carbonyle est de préférence porté par un carbocycle saturé ou insaturé, ayant de 5 ou 6 atomes de carbone.
Le ou les cycles de ce composé polycyclique peuvent porter des substituants. Le nombre de substituants sur chaque cycle est généralement de 1 à 3, de préférence 1 ou 2.
Comme exemples spécifiques de substituants on peut mentionner :

- un radical alkyle linéaire ou ramifié ayant de 1 à 6 atomes de carbone, de préférence un radical méthyle,
- un radical de formule

$$-\underset{\underset{CH_3}{|}}{C}=CH_2,$$

- un radical de formule $-CH_2-O-CH_3$,
- un atome d'halogène, de préférence, de chlore.

A titre illustratif, on peut citer plus particulièrement les composés de formule suivante :

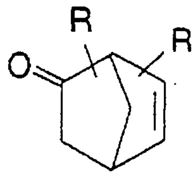 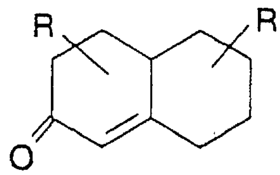 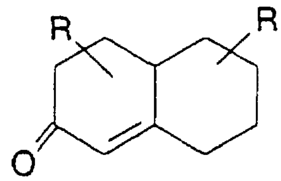

A peut représenter le reste d'un composé carbocyclique polycyclique, de préférence, bicyclique comprenant deux carbocycles insaturés, ayant chacun de préférence de 5 ou 6 atomes de carbone. Il peut y avoir présence d'un groupe carbonyle sur l'un des deux cycles.

Dans ces composés polycycliques, un ou plusieurs atomes de carbone, de préférence deux, peuvent être remplacés par un hétéroatome, de préférence un atome d'azote ou d'oxygène.

Le ou les cycles de ce composé polycyclique peuvent porter des substituants. Le nombre de substituants sur chaque cycle est généralement de 1 à 5, de préférence 1 ou 2.

Généralement, les substituants sont un ou plusieurs radicaux alkyle linéaire ou ramifié ayant de 1 à 6 atomes de carbone, de préférence un radical méthyle, éthyle.

A titre illustratif, on peut citer plus particulièrement les composés de formule suivante :

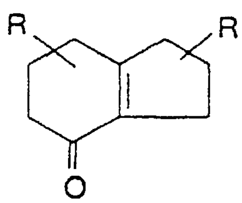 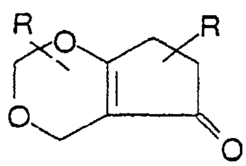 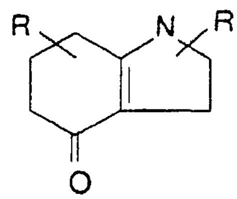 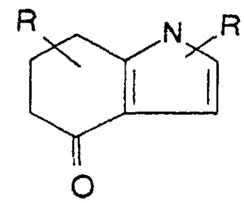

A peut représenter le reste d'un composé carbocyclique polycyclique comprenant au moins un carbocycle aromatique, de préférence, un cycle benzénique et un carbocycle ayant de préférence de 4 à 7 atomes de carbone et comprenant un ou deux groupes carbonyle.

A est de préférence le reste d'un composé bicyclique comprenant un cycle benzénique et un carbocycle de 5 ou 6 atomes de carbone comprenant un ou deux groupes carbonyle.

Les deux cycles de ce radical bicyclique peuvent porter des substituants. Le nombre de substituants sur chaque cycle est généralement de 1 ou 2.

Comme exemples spécifiques de substituants on peut mentionner :

- un radical alkyle linéaire ou ramifié ayant de 1 à 6 atomes de carbone, de préférence un radical méthyle, tert-butyle,
- un radical de formule

=C-CN,
 |
 CN

- un radical alkoxy linéaire ou ramifié ayant de 1 à 6 atomes de carbone, de préférence un radical méthoxy,
- un radical alkoxy linéaire ou ramifié ayant de 1 à 6 atomes de carbone porteur d'autres groupes fonctionnels tels que, par exemple, un groupe OH et/ou $N[R_{10}]_2$ avec $R_{10}$, identiques ou différents, représentant un atome d'hydrogène, un radical alkyle linéaire ou ramifié ayant de 1 à 6 atomes de carbone, de préférence un radical de formule $-O-CH_2-CHOH-CH_2-NHBu-t$
- un groupe OH,
- un groupe acyle ayant de 2 à 6 atomes de carbone, de préférence un radical acétyle ou de formule -CO-tert-butyle,
- un groupe $-CH_2-COOH$,
- un groupe $-NH_2$,
- un atome d'halogène, de préférence, de fluor, de chlore, de brome.

A titre illustratif, on peut citer plus particulièrement les composés de formule suivante :

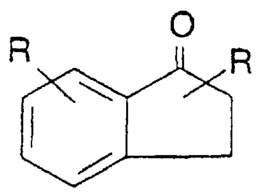 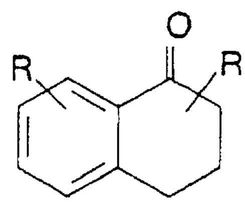 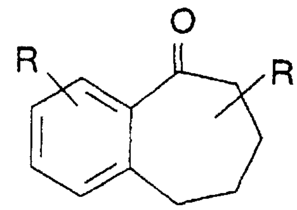

Parmi tous les composés cétoniques cycliques précités, le procédé de l'invention s'applique plus particulièrement, aux composés cétoniques carbocycliques saturés ou insaturés, ayant de 5 à 6 atomes de carbone dans le cycle et répondant à la formule (Ia) dans laquelle :

- R représente un atome d'hydrogène, un radical alkyle ou alkoxy linéaire ou ramifié, ayant de 1 à 6 atomes de carbone, de préférence, de 1 à 4 atomes de carbone, un radical benzylidène éventuellement porteur d'un atome d'halogène,
- n est égal à 1.

Les cétones cycliques répondant à la formule (Ia) mises en oeuvre préférentiellement dans le procédé de l'invention sont choisies parmi :

- celles dans lesquelles le reste A de la formule (Ia) représente le reste d'un composé monocyclique saturé comprenant un seul groupe carbonyle, telles que :
  - cyclobutanone,
  - cyclopentanone,
  - 2-méthylcyclopentanone,
  - 3-méthylcyclopentanone,
  - 2-méthyl-2-carboxyméthylcyclopentanone
  - 2,2-diméthylcyclopentanone,
  - 2-(2-octényl)-cyclopentanone,
  - 2-(3,7-diméthyl-2,6-octadiényl) cyclopentanone,
  - 2-cyclopentylidènecyclopentanone,
  - 2-benzylidènecyclopentanone,
  - 2-[(p-chloro)benzylidène]cyclopentanone,
  - 2-méthyl-2-carboxyméthyl-5-[(p-chloro)benzylidène]cyclopentanone
  - 2,4-diméthylcyclopentanone,
  - 2,5-diméthylcyclopentanone,
  - 3,4-diméthylcyclopentanone,
  - 2,2,4-triméthylcyclopentanone,
  - 4-méthylcyclohexanone,
  - 4-tert-butylcyclohexanone,
  - 5-méthyl-2-(1-méthyléthylidène)-cyclohexanone,
  - 6-cétoprostaglandine E1,
  - méthylester prostaglandine E2,
  - prostaglandine D2,
  - cyclohexanone,
  - 3-méthylcyclohexanone,
  - 4-méthylcyclohexanone,
  - 4-tert-butylcyclohexanone,
  - 4-n-pentylcyclohexanone,
  - 2-benzylidènecyclohexanone,
  - 2-(N,N,-diméthylamino)cyclohexanone,
  - 3,5-diméthylcyclohexanone,
  - dihydrocarvone,
  - cycloheptanone,
  - cyclooctanone,
  - cycloheptadécanone,

- celles dans lesquelles le reste A de la formule (Ia) représente le reste d'un composé monocyclique saturé comprenant deux groupes carbonyle, telles que :

    - 1,3-cyclopentanedione
    - 2-allyl-2-méthyl-1,3-cyclopentanedione,
    - 3,3-diméthyl-1,2-cyclopentanedione,
    - 3,4-diméthyl-1,2-cyclopentanedione,
    - 1,2-cyclohexanedione,
    - 1,3-cyclohexanedione,
    - 1,4-cyclohexanedione,
    - 1,2-cycloheptanedione,

- celles dans lesquelles le reste A de la formule (Ia) représente le reste d'un composé monocyclique insaturé comprenant un seul groupe carbonyle, telles que :

    - 2-cyclopenténone,
    - 3-méthyl-2-cyclopenténone,
    - 4,4-diméthyl-2-cyclopenténone,
    - 2-pentyl-2-cyclopenténone,
    - 3-éthoxy-2-cyclopenténone,
    - 2-hydroxy-3-éthyl-2-cyclopenténone,
    - prostaglandine J2,
    - jasmone,
    - 2-hydroxy-3,4-diméthyl-2-cyclopenténone,
    - 15-oxoprostaglandine E2,
    - 2-éthoxy-2-cyclohexénone,
    - 3-bromo-2-cyclohexénone,
    - carvone,
    - 8-hydroxycarvotanacétone,
    - 2-méthyl-5-(1-méthyléthènyl)-2-cyclohexénone,
    - 3,5,5-triméthyl-2-cyclohexénone,
    - méthyl ester de l'acide abscisique,
    - 2-hydroxy-3-méthyl-6-(1 -méthyléthyl)-2-cyclohexénone,
    - 5-cyclohexadécènone.

- celles dans lesquelles le reste A de la formule (Ia) représente le reste d'un composé monocyclique insaturé comprenant deux groupes carbonyle, telles que :

    - 2-cyclopentène-1,4-dione,
    - 4-hydroxy-5-méthyl-4-cyclopentène-1,3-dione,

- celles dans lesquelles le reste A de la formule (Ia) représente le reste d'un composé bicyclique saturé comprenant un ou deux groupes carbonyle, telles que :

    - isobornylcyclohexanone,
    - isofenchylcyclohexanone,
    - isocamphylcyclohexanone,
    - bomylcyclohexanone,
    - fenchylcyclohexanone,
    - camphylcyclohexanone,
    - camphor,
    - norcamphor,
    - 3-bromocamphor,
    - 2,3-bornanedione,
    - 1-décalone,
    - 2-décalone,
    - N-(éthoxycarbonyl)nortropinone,

- celles dans lesquelles le reste A de la formule (Ia) représente le reste d'un composé bicyclique saturé/insaturé comprenant un ou deux groupes carbonyle, telles que :

  - bicyclo[3.2.0]hept-2-en-6-one,
  - 1-(méthoxyméthyl)-bicyclo[2.2.0]hept-5-en-2-one,
  - 3,4,8,8a-tétrahydro-8a-méthyl-1,6(2H,7H)-naphtalènedione.

- celles dans lesquelles le reste A de la formule (Ia) représente le reste d'un composé bicyclique insaturé comprenant un groupe carbonyle, telles que :

  - 6,7-dihydro-cyclopenta-1,3-dioxin-5(4H)-one
  - 6,7-dihydro-1,1,2,3,3-pentaméthyl-4(5H)-indanone,
  - 4-oxo-4,5,6,7-tétrahydroindole.

- celles dans lesquelles le reste A de la formule (Ia) représente le reste d'un composé bicyclique dont l'un d'eux est aromatique comprenant un ou deux groupes carbonyle, telles que :

  - 2-indanone,
  - 2-méthyl-1-indanone,
  - 4-méthyl-1-indanone,
  - 4-méthoxy-1-indanone,
  - 6-méthoxy-1-indanone,
  - 4-hydroxy-1-indanone,
  - 5-bromo-1-indanone,
  - 1,3-indanedione,
  - 1-tétralone,
  - 2-tétralone,
  - 4-méthyl-1 -tétralone,
  - 5,7-diméthyl-1-tétralone,
  - 5-méthoxy-1-tétralone,
  - 6,7-diméthoxy-1-tétralone,
  - 5-hydroxy-1-tétralone,
  - levobunolol.

Parmi tous les composés de formule (I) susceptibles d'être mis en oeuvre dans le procédé de l'invention, on fait appel plus particulièrement aux composés suivants :

- benzaldéhyde,
- anisaldéhyde,
- 4-chlorobenzaldéhyde,
- aldéhyde vératrique,
- aldéhyde cinnamique,
- $\alpha$-naphtylaldéhyde,
- isobornylcyclohexanone,
- isofenchylcyclohexanone,
- isocamphylcyclohexanone,
- bornylcyclohexanone,
- fenchylcyclohexanone,
- camphylcyclohexanone,
- 4-méthylcyclohexanone,
- 4-tert-butylcyclohexanone.

Pour ce qui est de l'alcool mis en oeuvre, il s'agit d'un composé qui permet le transfert de l'hydrure sur le groupe carbonyle.

Il répond plus particulièrement à la formule (III) :

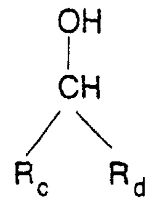

(III)

dans ladite formule (III) :

- $R_c$ et $R_d$ ont la signification donnée précédemment pour $R_a$ et $R_b$ dans la formule (I).

Il s'agit préférentiellement d'un alcool aliphatique ou cycloaliphatique, secondaire ou tertiaire.

Etant donné que ce réactif est consommé au cours de la réaction, il y a intérêt qu'il soit le moins cher possible. C'est pourquoi l'on préfère faire appel à des alcools ayant une faible condensation en carbone, de préférence, moins de 6 atomes de carbone et encore plus préférentiellement moins de 4 atomes de carbone.

Comme exemples plus spécifiques d'alcools convenant à la mise en oeuvre de l'invention, on peut citer entre autres, l'isopropanol, l'isobutanol, le sec-butanol, le tert-butanol, le glycérol.

L'isopropanol est l'alcool mis en oeuvre préférentiellement.

Conformément à l'invention, la réaction de réduction est conduite en présence d'un catalyseur zéolithique.

Par "zéolithe", on entend un tectosilicate cristallisé d'origine naturelle ou synthétique dont les cristaux résultent de l'assemblage tridimensionnel d'unités tétraédriques de $SiO_4$ et $TO_4$ : T représentant un élément trivalent tel que aluminium, gallium, bore, fer, de préférence, l'aluminium.

Les zéolithes de type aluminosilicate sont les plus communes.

Les zéolithes présentent au sein du réseau cristallin, un système de cavités reliées entre elles par des canaux d'un diamètre bien défini que l'on appelle les pores.

Les zéolithes peuvent présenter un réseau de canaux monodimensionnel, bidimensionnel ou tridimensionnel.

Dans le procédé de l'invention, on peut faire appel à une zéolithe naturelle ou synthétique.

Comme exemples de zéolithes naturelles susceptibles d'être utilisées, on peut citer, par exempte : la chabazite, la clinoptilolite, l'érionite, la phillipsite, l'offrétite.

Conviennent tout à fait bien à la mise en oeuvre de l'invention, les zéolithes synthétiques.

Comme exemples de zéolithes synthétiques à réseau monodimensionnel, on peut citer entre autres, la zéolithe ZSM-4, la zéolithe L, la zéolithe ZSM-12, la zéolithe ZSM-22, la zéolithe ZSM-23, la zéolithe ZSM-48.

A titre d'exemples de zéolithes à réseau bidimensionnel mises en oeuvre préférentiellement, on peut mentionner la mordénite, la ferrierite.

En ce qui concerne les zéolithes à réseau tridimensionnel, on peut nommer plus particulièrement, la zéolithe $\beta$, la zéolithe $\beta$ au titane, la zéolithe Y, la zéolithe X, la zéolithe ZSM-5, la zéolithe ZSM-11, l'offrétite.

On fait appel préférentiellement aux zéolithes synthétiques et plus particulièrement aux zéolithes qui sont sous les formes suivantes :

- la mazzite de rapport molaire Si/Al de 3,4,
- la zéolithe L de rapport molaire Si/Al de 1,5 à 3,5,
- la mordénite de rapport molaire Si/Al de 5 à 15,
- la ferrierite de rapport molaire Si/Al de 3 à 10,
- l'offrétite de rapport molaire Si/Al de 4 à 8,5.
- les zéolithes $\beta$ de rapport molaire Si/Al supérieur à 8, généralement compris entre 10 et 100, de préférence, entre 12 et 50, et encore plus préférentiellement entre 12 et 35,
- les zéolithes $\beta$ au titane de rapport Si/Al supérieur à 50, de préférence, compris entre 200 et 600 et la teneur en Ti exprimée en % en poids de $TiO_2$ varie entre 0,1 et 10 %, de préférence, entre 1 et 5 %,
- les zéolithes $\beta$ au titane sans aluminium ou à très faible teneur (rapport Si/Al supérieur à 100 000) ; la teneur en Ti exprimée en % en poids de $TiO_2$ varie entre 0,1 et 10 %, de préférence, entre 1 et 5 %,
- les zéolithes Y en particulier les zéolithes obtenues après traitement de désalumination (par exemple hydrotraitement, lavage à l'aide d'acide chlorhydrique ou traitement par $SiCl_4$) et l'on peut citer plus particulièrement les zéolithes US-Y de rapport molaire Si/Al supérieur à 2, de préférence compris entre 6 et 60 ;
- la zéolithe X de type faujasite de rapport molaire Si/Al de 0,7 à 1,5,
- les zéolithes ZSM-5 ou silicalite d'aluminium de rapport molaire Si/Al de 10 à 500,
- la zéolithe ZSM-11 de rapport molaire Si/Al de 5 à 30,
- la zéolithe mésoporeuse de type MCM, plus particulièrement MCM-22 et MCM-41 de rapport molaire Si/Al compris

entre 10 et 100, de préférence, compris entre 15 et 40,

Parmi toutes ces zéolithes, on fait appel préférentiellement dans le procédé de l'invention aux zéolithes β et Y.

Quelle que soit la nature de la zéolithe, il peut être nécessaire afin d'obtenir le rapport Si/Al souhaité, de faire un traitement de désalumination.

Ainsi, on peut mettre en oeuvre les méthodes connues de l'homme du métier parmi lesquelles on peut citer, à titre d'exemples, et de manière non exhaustive, les calcinations en présence de vapeur, les calcinations en présence de vapeur d'eau suivies d'attaques par des acides minéraux ($HNO_3$, HCl...), les traitements directes de désalumination par des réactifs tels que le tétrachlorure de silicium ($SiCl_4$), l'hexafluorosilicate d'ammonium ($(NH_4)_2SiF_6$), l'éthylènediaminetétracétique (EDTA) ainsi que sa forme mono- ou disodique. On peut également faire un traitement de désalumination par attaque acide directe par des solutions d'acides minéraux tels que par exemple, l'acide chlorhydrique, l'acide nitrique, l'acide sulfurique ou d'acides organiques comme notamment l'acide acétique, l'acide oxalique.

Par ailleurs, toute combinaison des méthodes de désalumination précitées est aussi possible.

Une variante de l'invention consiste à mettre en oeuvre une zéolithe ayant subi une activation par calcination. L'opération de calcination est conduite à une température comprise entre 200°C et 800°C, de préférence entre 400°C et 700°C, pendant une durée variant de 1 à 24 heures, de préférence de 5 à 8 heures

Les zéolithes mises en oeuvre dans le procédé de l'invention, sont des produits connus décrits dans la littérature [cf. Atlas of zeolites structure types by W. M. Meier and D. H. Olson published by the Structure Commission of the International Zeolite Association (1978)].

On peut faire appel aux zéolithes disponibles dans le commerce ou bien les synthétiser selon les procédés décrits dans la littérature.

On peut se référer à l'Atlas précité, et plus particulièrement, pour la préparation :

- de la zéolithe L à la publication de Barrer R. M. et al, Z. Kristallogr., 128, pp. 352 (1969)
- de la zéolithe ZSM-12, au brevet US 3 832 449 et à l'article LaPierre et al, Zeolites 5, pp. 346 (1985),
- de la zéolithe ZSM-22, à la publication Kokotailo G.T. et al, Zeolites 5, pp. 349 (1985),
- de la zéolithe ZSM-23, au brevet US 4 076 842 et à l'article Rohrman A. C. et al, Zeolites 5, pp. 352 (1985),
- de la zéolithe ZSM-48, aux travaux de Schlenker J. L. et al, Zeolites 5, pp. 355 (1985),
- de la zéolithe β, au brevet US 3 308 069 et à l'article Caullet P. et al, Zeolites 12, pp. 240 (1992),
- de la zéolithes β au titane, aux brevets FR 2 730 723, FR 2 730 722 et WO 97/33830,
- de la mordénite, aux travaux de Itabashi et al, Zeolites 6, pp. 30 (1986),
- des zéolithes X et Y respectivement aux brevets US 2 882 244 et US 3 130 007,
- de la zéolithe ZSM-5, au brevet US 3 702 886 et à l'article Shiralkar V. P. et al, Zeolites 9, pp. 363 (1989),
- de la zéolithe ZSM-11, aux travaux de Harrison I. D. et al, Zeolites 7, pp. 21 (1987).
- la zéolithe mésoporeuse de type MCM, à l'article de Beck et al, J. Am. Chem. Soc. 114 (27), pp. 10834-43 (1992).

La zéolithe constitue la phase catalytique. Elle peut être utilisée seule ou en mélange avec une matrice minérale. Dans la description, on désignera par "catalyseur", le catalyseur réalisé entièrement en zéolithe ou en mélange avec une matrice préparée selon des techniques connues de l'Homme du métier.

A cet effet, la matrice peut être choisie parmi les oxydes de métaux, tels que les oxydes d'aluminium, de silicium et/ou de zirconium, ou encore parmi les argiles et plus particulièrement, le kaolin, le talc ou la montmorillonite.

Dans le catalyseur, la teneur en phase active représente de 5 à 100 % du poids du catalyseur.

Les catalyseurs peuvent se présenter sous différentes formes dans le procédé de l'invention : poudre, produits mis en forme tels que granulés (par exemple, extrudés ou billes), pastilles, qui sont obtenus par extrusion, moulage, compactage ou tout autre type de procédé connu. En pratique, sur le plan industriel, ce sont les formes de granulés ou de billes qui présentent le plus d'avantages tant sur le plan de l'efficacité que sur le plan de commodité de mise en oeuvre.

Conformément à l'invention, la réaction de réduction est conduite selon un procédé de recirculation du mélange réactionnel sur lit fixe de catalyseur.

On commence par effectuer le mélange d'une façon quelconque du composé carbonylé et de l'alcool.

Ainsi, on peut mélanger le composé carbonylé et l'alcool dans une zone de mélange, puis envoyer le mélange obtenu sur le lit catalytique.

Selon une autre variante, on peut introduire l'un des réactifs (composé carbonylé ou alcool), et l'envoyer sur le lit catalytique puis ajouter l'autre réactif, en une seule fois ou progressivement lorsque la température réactionnelle souhaitée est atteinte. Dans ce mode, préférentiellement, on introduit le composé carbonylé, puis l'on ajoute progressivement l'alcool.

On ne sortira pas du cadre de l'invention, en introduisant un mélange de réactifs puis à ajouter, à la température souhaitée, l'un des deux autres réactifs, de telle sorte que l'on obtienne le ratio alcool/composé carbonylé désiré.

Le rapport final entre le nombre de moles d'alcool et le nombre de moles de composé carbonylé peut varier largement. Ainsi, le rapport peut aller de 0,1 à 20, et se situe de préférence entre 0,5 et 4,0

Un mode de réalisation préférée de l'invention consiste, afin d'accroître le rendement en maintenant l'activité du catalyseur, de mettre en oeuvre un excès d'alcool. Ainsi, on choisit un rapport molaire alcool/composé carbonylé égal à au moins 1, de préférence, compris entre 1 et 20 et encore plus préférentiellement entre 1 et 10.

L'un des réactifs est généralement utilisé comme solvant réactionnel mais l'invention n'exclut pas non plus la mise en oeuvre d'un solvant organique dont la nature est déterminée par l'Homme du métier.

Il est à noter qu'il est souhaitable de minimiser la quantité d'eau présente lors de la réaction. Ainsi, elle est au plus égale à environ 10 % du poids de substrat (composé carbonylé) et de préférence égale à au plus 1 % du poids du substrat. La réaction est conduite préférentiellement en l'absence d'eau.

Selon un mode de réalisation préféré du procédé de l'invention, on porte la température du mélange à la température à laquelle est conduite la réaction.

La température à laquelle est mise en oeuvre la réaction de réduction dépend de la réactivité du substrat de départ et de celle de l'alcool.

Elle se situe entre 20°C et 200°C, de préférence entre 40°C et 150°C.

On fait passer les réactifs sur un lit catalytique comprenant au moins une zéolithe.

La quantité de catalyseur que l'on met en oeuvre dans le procédé de l'invention peut varier dans de larges limites.

Le catalyseur peut représenter en poids par rapport au composé carbonylé engagé, de 0,01 à 50 %, de préférence, de 1,0 à 20 %.

Généralement, la réaction est conduite à pression atmosphérique mais des pressions plus faibles ou plus élevées peuvent également convenir. On travaille sous pression autogène lorsque la température de réaction est supérieure à la température d'ébullition des réactifs et/ou des produits.

Le mélange réactionnel traverse le lit catalytique, de préférence, de bas en haut et à sa sortie, est renvoyé dans la zone de mélange des réactifs afin d'être recyclé un nombre de fois suffisant pour obtenir le taux de conversion du substrat souhaité, de préférence, supérieure à 20 %, et encore plus préférentiellement compris entre 50 et 100 %. On définit le taux de conversion du substrat comme le rapport entre le nombre de moles de substrat transformées sur le nombre de moles de substrat introduites.

La vitesse linéaire du flux liquide sur le lit catalytique varie avantageusement entre 0,1 et 10 cm/s, de préférence, entre 0,1 et 5 cm/s.

Le temps de séjour du flux de matière sur le lit catalytique varie, par exemple, entre 15 min et 15 h, et de préférence, entre 30 min et 10 h.

En fin de réaction, on obtient une phase liquide comprenant un alcool qui peut être récupéré de manière classique, par distillation ou par recristallisation dans un solvant approprié, après élimination préalable des réactifs en excès.

Pour une meilleure compréhension de l'invention, on a représenté sur la figure 1 annexée un mode de réalisation préférée de l'invention.

Dans le réacteur (1), on effectue le mélange du composé carbonylé, de préférence, une cétone et de l'alcool. Il s'agit d'un réacteur agité ou non, muni de vannes d'arrivée des réactifs et de vidange et équipé d'un dispositif de chauffage ou pourvu d'une double enveloppe permettant le chauffage du mélange par circulation d'un liquide à température adéquate. L'agitation qui n'est pas obligatoire peut être réalisée grâce à un agitateur Impeller® (2).

Dans le réacteur (1), sont introduits le composé carbonylé, de préférence, une cétone (3) et l'alcool (4).

Le mélange réactionnel est envoyé par l'intermédiaire de tout moyen approprié, notamment une pompe centrifuge (5), en (6) en pied d'un réacteur tubulaire (7) comportant le catalyseur zéolithique solide disposé en lit fixe (8).

En sortie de réacteur (9), le mélange réactionnel est renvoyé par l'intermédiaire d'un conduit (10) dans le réacteur (1) et circule ainsi en boucle fermée.

En fin de réaction, on récupère le mélange réactionnel par vidange du mélangeur (1) par l'intermédiaire d'une vanne (11) non symbolisée sur le schéma.

Le procédé de l'invention est particulièrement bien adapté à la préparation de mélanges d'isomères cis ou trans de cyclohexanol substitué, de préférence en position para par rapport au groupe hydroxyle, comprenant au moins 90 %, de préférence, au moins 95 % de l'isomère cis ou trans : le substituant étant préférentiellement un groupe alkyle aliphatique ou cycloaliphatique, ayant de 1 jusqu'à 12 atomes de carbone, de préférence, de 1 à 4.

L'invention convient tout à fait bien à la préparation de mélanges d'isomères de 4-méthylcyclohexanol ou 4-tert-butylcyclohexanol, comprenant au moins 90 %, de préférence, au moins 95 % de l'isomère cis.

Les exemples qui suivent, illustrent l'invention sans toutefois la limiter.

Dans les exemples, les rendements mentionnés correspondent à la définition suivante :

$$\text{Rendement : } RR_{A.A.} = \frac{\text{nombre de moles d'alcool formées}}{\text{nombre de moles de cétone introduites}} \%$$

EXEMPLES

On donne ci-après, le protocole opératoire qui est suivi dans les différents exemples. On se réfère à la figure 1.
Dans un réacteur inox (7), on charge le catalyseur zéolithique pour former le lit catalytique qui repose sur une épaisseur (- 20 mm) de billes de verre ainsi que le haut du lit qui est recouvert d'environ 20 mm de billes de verre de diamètre 5 mm.
Dans un réacteur (1) Sovirel ® double enveloppe de 3 litres, on charge la cétone (3) et l'isopropanol (4), à température ambiante.
On agite (environ 500 tours/min) et l'on obtient une solution homogène.
On chauffe cette solution au reflux et on fait circuler cette solution sur le lit fixe à un débit de 60 l/h.
Le débit est maintenu pendant toute la durée de la réaction.
En fin de réaction, on coupe la recirculation sur le lit fixe et on laisse refroidir le mélange réactionnel.
Le mélange réactionnel est ensuite distillé pour récupérer le solvant et les alcools formés.

Exemple 1

Dans cet exemple, on effectue la réduction de la 4-méthylcyclohexanone.
On met en oeuvre un catalyseur comprenant 40 % de liant (alumine) et 60 % d'une zéolithe β commercialisée par la Société PQ.
La zéolithe utilisée est une zéolithe de rapport "Si/Al" de 12,5,
**[0153]** On l'engage à raison de 20 % en poids par rapport à la 4-méthylcyclohexanone, soit 92 g.
Le nombre de mole d'isopropanol engagé est 8 fois supérieur à celui de la cétone.
La température de la réaction est de 80°C.
On détermine par chromatographie en phase gazeuse un rendement (RR) de 99,6 % en alcools cyclohexaniques après 5 h de réaction et une sélectivité (RT) en cis 4-méthylcyclohexanol de 96,8 %.

Exemples 2 à 3

Sur le même lit catalytique que précédemment, on répète cette opération, 2 fois.
Pour obtenir les mêmes résultats que précédemment, les temps de réaction sont respectivement de 8 h et 12 h 30.

Exemple 4

On utilise le catalyseur utilisé précédemment après avoir été calciné 18 h à 500°C.
Après 5 h 15 de réaction, on détermine par chromatographie en phase gazeuse, un rendement (RR) de 99,5 % en alcools cyclohexaniques et une sélectivité (RT) de 95,8 % en cis 4-méthylcyclohexanol.

Exemple 5

Dans cet exemple, on effectue la réduction de la 4-tert-butylcyclohexanone.
On met en oeuvre le même catalyseur qu'à l'exemple 1.
On l'engage à raison de 25 % en poids par rapport à la 4-tert-butylcyclohexanone, soit 105 g.
Le nombre de mole d'isopropanol engagé est 10 fois supérieur à celui de la cétone.
La température est identique à celle de l'exemple 1.
On détermine par chromatographie en phase gazeuse un rendement (RR) en alcools 4-tert-butylcyclohexaniques de 99,1 % et une sélectivité (RT) en cis 4-tert-butylcyclohexanol de 97,1 %.

Exemple 6

On reproduit l'exemple 1 mais en utilisant une zéolithe commercialisée par la Société PQ. qui est une zéolithe extrudée HY contenant 40% d'alumine comme liant : le rapport Si/Al est de 10.
Dans ce cas, le rendement en alcools cyclohexaniques est de 98 % et la sélectivité en trans 4-méthylcyclohexanol est de 92 %.

Exemple 7

On reproduit l'exemple 1 mais en utilisant une zéolithe mésoporeuse MCM-22 : le rapport Si/Al est de 14.
Dans ce cas, le rendement en alcools cyclohexaniques est de 85 % et la sélectivité en trans 4-méthylcyclohexanol est de 86 %.

Exemple 8

On reproduit l'exemple 1 mais en utilisant une zéolithe β au titane contenant 2,5 % de titane (exprimé en $TiO_2$) et ne contenant pas d'aluminium. Cette zéolithe est obtenue selon l'enseignement de WO 97/33830.
Dans ce cas, le rendement en alcools cyclohexaniques est de 95 % et la sélectivité en cis 4-méthylcyclohexanol est de 98 %.

## Revendications

**1.** Procédé de réduction d'un composé carbonylé en alcool correspondant, par réaction dudit composé carbonylé avec un alcool en présence d'un catalyseur zéolithique **caractérisé par le fait qu'**il consiste :

- à effectuer le mélange d'une manière quelconque du composé carbonylé et de l'alcool,
- à faire passer ledit mélange sur un lit catalytique comprenant au moins une zéolithe,
- à soumettre le mélange réactionnel issu du lit catalytique à une recirculation sur lit catalytique, en un nombre de fois suffisant pour obtenir le taux de conversion du substrat souhaité.

**2.** Procédé selon la revendication 1 **caractérisé par le fait que** le composé carbonylé répond à la formule générale (I) :

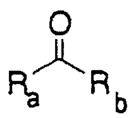
(I)

dans ladite formule (I) :

- $R_a$ et $R_b$, identiques ou différents, représentent un atome d'hydrogène ou un radical hydrocarboné monovalent, éventuellement substitué ayant de 1 à 40 atomes de carbone ; au plus l'un des radicaux $R_a$ ou $R_b$ est un atome d'hydrogène,
- $R_a$ et $R_b$ peuvent former un cycle comprenant éventuellement un autre hétéroatome.

**3.** Procédé selon l'une des revendications 1 et 2 **caractérisé par le fait que** le composé carbonylé répond à la formule générale (I) dans laquelle $R_a$ et $R_b$ représentent un radical hydrocarboné monovalent, substitué ou non qui peut être un radical aliphatique acyclique saturé ou insaturé, linéaire ou ramifié ; un radical carbocyclique ou hétérocyclique saturé, insaturé ou aromatique, monocyclique ou polycyclique.

**4.** Procédé selon l'une des revendications 2 et 3 **caractérisé par le fait que** le composé carbonylé répond à la formule générale (I) dans laquelle $R_a$ et $R_b$ représentent un radical aliphatique acyclique, saturé ou insaturé, linéaire ou ramifié ayant de préférence de 1 à 12 atomes de carbone, saturé ou comprenant une à plusieurs insaturations sur la chaîne, généralement, 1 à 3 insaturations qui peuvent être des doubles liaisons simples ou conjuguées ou des triples liaisons.

**5.** Procédé selon l'une des revendications 2 à 4 **caractérisé par le fait que** le composé carbonylé répond à la formule générale (I) dans laquelle $R_a$ et $R_b$ représentent un radical alkyle, alcényle, alcadiényle, linéaire ou ramifié ayant de préférence de 1 à 12 atomes de carbone : la chaîne hydrocarbonée pouvant être éventuellement :

- interrompue par l'un des groupes suivants Z :
  -O- ; -CO- ; COO- ; $-NR_1-$ ; $-CO-NR_1-$; -S- ; $-SO_2-$ ; $-NR_1-CO-$ ; dans lesdites formules $R_1$ représente un atome d'hydrogène, un groupe alkyle linéaire ou ramifié ayant de 1 à 6 atomes de carbone, de préférence,

un radical méthyle ou éthyle,

- et/ou porteuse de l'un des substituants suivants :
  - OH ; -COOH ; -COOX ; -CO-N($R_1$)($R_2$) ; -COO$R_1$ ; -CHO ; -CO$R_1$ ; -$NO_2$ ; -X ; -$CF_3$.

dans ces formules, X représente un atome d'halogène, de préférence, un atome de chlore ou de brome et $R_2$ a la même signification que $R_1$ donnée précédemment.

**6.** Procédé selon l'une des revendications 2 à 5 **caractérisé par le fait que** le composé carbonylé répond à la formule générale (I) dans laquelle $R_a$ et $R_b$ représentent un radical aliphatique acyclique, saturé ou insaturé, linéaire ou ramifié porteur d'un substituant cyclique, de préférence, un cycle benzénique : le radical aliphatique acyclique pouvant être relié au cycle par un lien valentiel ou par l'un des groupes Z précités dans la revendication 5.

**7.** Procédé selon l'une des revendications 2 et 3 **caractérisé par le fait que** le composé carbonylé répond à la formule générale (I) dans laquelle $R_a$ et $R_b$ représentent :

- un radical carbocyclique ou hétérocyclique, monocyclique, saturé ou comprenant 1 ou 2 insaturations dans le cycle ; le nombre d'atomes de carbone dans le cycle pouvant varier de 3 à 8 atomes de carbone et étant de préférence égal à 5 ou 6,
- un radical carbocyclique ou hétérocyclique, polycyclique, de préférence bicyclique ; le nombre d'atomes de carbone dans le cycle pouvant varier de 3 à 8 atomes de carbone et étant de préférence égal à 5 ou 6.

**8.** Procédé selon l'une des revendications 2 et 3 **caractérisé par le fait que** le composé carbonylé répond à la formule générale (I) dans laquelle $R_a$ et $R_b$ représentent un radical carbocyclique aromatique, et notamment benzénique répondant à la formule générale (II) :

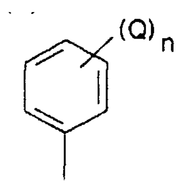

(II)

dans ladite formule (II) :

- n est un nombre entier de 0 à 5, de préférence de 0 à 3,
- Q représente $R_3$, l'un des groupes ou fonctions suivantes :
  - . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  - . un radical alcényle linéaire ou ramifié ayant de 2 à 6 atomes de carbone, de préférence, de 2 à 4 atomes de carbone, tel que vinyle, allyle,
  - . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  - . un groupe acyle ayant de 2 à 6 atomes de carbone,
  - . un radical de formule :

$$-R_5-OH$$

$$-R_5-COOR_7$$

$$-R_5-CHO$$

$$-R_5-NO_2$$

$-R_5-CN$

$-R_5-N(R_7)(R_8)$

$-R_5-CO-N(R_7)(R_8)$

$-R_5-SH$

$-R_5-X$

$-R_5-CF_3$

dans lesdites formules, $R_5$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; les radicaux $R_7$ et $R_8$, identiques ou différents, représentent un atome d'hydrogène ou un radical alkyle linéaire ou ramifié ayant de 1 à 6 atomes de carbone ; X symbolise un atome d'halogène, de préférence un atome de chlore, de brome ou de fluor.

. Q représente $R_4$, l'un des radicaux plus complexes suivants :

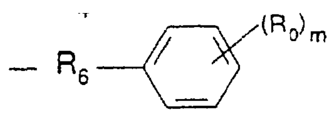

dans lequel :

- m est un nombre entier de 0 à 5, de préférence de 0 à 3,

  . $R_0$ ayant la signification donnée précédemment pour $R_3$,
  . $R_6$ représente un lien valentiel ; un groupe hydrocarboné divalent, linéaire ou ramifié, saturé ou insaturé ayant de 1 à 6 atomes de carbone tel que par exemple, méthylène, éthylène, propylène, isopropylène, isopropylidène ou l'un des groupes suivants dénommés Z :
  -O- ; -CO- ; COO- ; $-NR_7-$ ; $-CO-NR_7-$ ; -S- ; $-SO_2-$ ; $-NR_7-CO-$;

dans lesdites formules $R_7$ représente un atome d'hydrogène, un groupe alkyle linéaire ou ramifié ayant de 1 à 6 atomes de carbone, de préférence, un radical méthyle ou éthyle.

**9.** Procédé selon l'une des revendications 2 et 3 **caractérisé par le fait que** le composé carbonylé répond à la formule générale (I) dans laquelle $R_a$ et $R_b$ représentent :

- un radical hétérocyclique aromatique comportant 5 ou 6 atomes dans le cycle dont 1 ou 2 hétéroatomes,
- un radical carbocyclique ou hétérocyclique aromatique polycyclique.

**10.** Procédé selon l'une des revendications 2 et 3 **caractérisé par le fait que** le composé carbonylé répond à la formule générale (I) dans laquelle $R_a$ représente un radical phényle éventuellement porteur d'un groupe alkyle ou alkoxy ayant de 1 à 4 atomes de carbone, un groupe trihalogénométhyle ou un atome d'halogène et $R_b$, un atome d'hydrogène.

**11.** Procédé selon l'une des revendications 1 et 2 **caractérisé par le fait que** le composé carbonylé répond à la formule générale (Ia) :

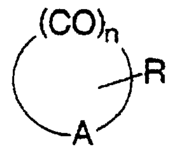

(Ia)

dans laquelle :

- A symbolise le reste d'un cycle formant tout ou partie d'un système monocyclique ou polycyclique comprenant au moins un groupe carbonyle,
- R représente un atome d'hydrogène ou un ou plusieurs substituants, identiques ou différents,
- n est un nombre de préférence égal à 1 ou 2.

**12.** Procédé selon l'une des revendications 1 et 2 **caractérisé par le fait que** le composé carbonylé répond à la formule (Ia) dans laquelle le reste A éventuellement substitué représente le reste :

- d'un composé monocyclique, carbocyclique, saturé ou insaturé,
- d'un composé polycyclique comprenant au moins deux carbocycles, saturés et/ou insaturés,
- d'un composé polycyclique comprenant au moins deux cycles saturés et/ou insaturés : un ou plusieurs des atomes de carbone pouvant être remplacés par un hétéroatome,
- d'un composé polycyclique comprenant au moins deux carbocycles dont l'un d'eux est aromatique.

**13.** Procédé selon l'une des revendications 1 et 2 **caractérisé par le fait que** le composé carbonylé est un composé monocyclique carbocyclique avec un nombre d'atomes de carbone dans le cycle variant entre 3 et 20 atomes de carbone, de préférence égal à 5 ou 6 atomes de carbone ; ledit carbocycle comprenant éventuellement 1 à 2 doubles liaisons.

**14.** Procédé selon l'une des revendications 1 et 2 **caractérisé par le fait que** le composé carbonylé est un composé polycyclique avec un nombre d'atomes de carbone dans chaque cycle variant entre 3 et 8 atomes de carbone, de préférence égal à 5 ou 6 atomes de carbone.

**15.** Procédé selon l'une des revendications 1 et 2 **caractérisé par le fait que** le composé carbonylé est un composé polycyclique comprenant au moins deux cycles saturés et/ou insaturés : un ou deux des atomes de carbone pouvant être remplacés par un hétéroatome, de préférence un atome d'oxygène ou d'azote.

**16.** Procédé selon l'une des revendications 11 à 15 **caractérisé par le fait que** le composé carbonylé répond à la formule (Ia) dans laquelle le reste A porte un ou plusieurs substituants R tels que :

- R peut représenter $R_9$, l'un des groupes suivants :

  . un radical aliphatique acyclique, linéaire ou ramifié, ayant de 1 à 20 atomes de carbone, saturé ou comprenant une ou plusieurs insaturations sur la chaîne, de préférence 1 à 3 insaturations qui sont de préférence, des doubles liaisons simples ou conjuguées ; la chaîne hydrocarbonée pouvant être éventuellement :

  . interrompue par l'un des groupes suivants dénommés Z :
    -O- ; -CO- ; COO- ; $-NR_{10}-$ ; $-CO-NR_{10}-$ ; -S- ; $-SO_2-$ ;
  dans lesdites formules $R_{10}$ représente un atome d'hydrogène, un radical alkyle linéaire ou ramifié ayant de 1 à 6 atomes de carbone,
  . et/ou porteuse de l'un des substituants suivants :
    -OH ; -CN ; $-N[R_{10}]_2$ ; $-COOR_{10}$ ; $-CF_3$ ou -X ;
  dans lesdites formules, les radicaux $R_{10}$, identiques ou différents, représentent un atome d'hydrogène, un radical alkyle linéaire ou ramifié ayant de 1 à 6 atomes de carbone et X représente, un atome d'halogène, de préférence, de fluor, de chlore, de brome,
  . un radical du type $=R_{11}$, dans lequel $R_{11}$ représente un radical alkylidène ayant de 1 à 6 atomes de carbone, un radical de formule $=C(CN)_2$ ou un radical cycloalkylidène ou cycloalcénylidène ayant 5 ou 6 atomes de carbone ou un radical benzylidène éventuellement substitué, de préférence par un atome d'halogène X,

. un radical alkoxy linéaire ou ramifié ayant de 1 à 6 atomes de carbone,
. deux atomes successifs du cycle peuvent être reliés entre eux par un pont époxy ou par un pont du type alkylène dioxy ayant de 1 à 4 atomes de carbone, de préférence les radicaux méthylène dioxy, éthylène dioxy, propylène dioxy,
. un groupe OH,
. un groupe $COOR_{10}$, $R_{10}$ représentant un atome d'hydrogène ou un radical alkyle ayant de 1 à 6 atomes de carbone, de préférence méthyle, éthyle,
. un groupe CN,
. un atome d'halogène, de préférence, de fluor, de chlore, de brome.
. un groupe $-CF_3$.

- R peut représenter $R_{12}$, l'un des groupes plus complexes suivants :

. un radical carbocyclique saturé ou insaturé ayant de 4 à 7 atomes de carbone, de préférence un radical cyclopentyle, cyclohexyle, cyclopentène-2 yle, cyclopentène-3 yle, cyclohexène-1 yle, cyclohexène-2 yle, cyclohexène-3 yle,
. un radical de formule

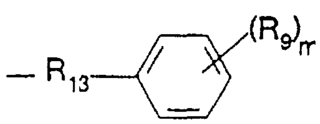

dans lequel $R_{13}$ représente un lien valentiel ou un radical hydrocarboné divalent, linéaire ou ramifié, saturé ou insaturé ayant de 1 à 6 atomes de carbone tel que par exemple, méthylène, éthylène, propylène, isopropylène, isopropylidène et $R_9$ ayant la signification donnée précédemment et m est un nombre entier de 0 à 4,
. un radical - $R_{13}$ - Z - $R_{14}$ dans lequel Z et $R_{13}$ ont la signification donnée précédemment, $R_{14}$ représente un radical alkyle linéaire ou ramifié ayant de 1 à 6 atomes de carbone ou un radical de formule

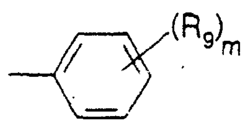

dans lequel $R_9$ et m ont la signification donnée précédemment,

. un radical de type spiro de formule :

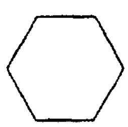
ou
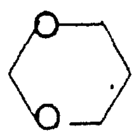
ou
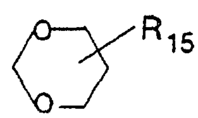

dans laquelle $R_{15}$ représente un ou plusieurs radicaux alkyle, linéaires ou ramifiés ayant de 1 à 6 atomes de carbone.

**17.** Procédé selon l'une des revendications 11 à 13 **caractérisé par le fait que** le composé carbonylé répond à la formule (Ia) dans laquelle A est le reste d'un composé carbocyclique monocyclique, saturé portant un ou plusieurs substituants tels que :

. un radical alkyle linéaire ou ramifié ayant de 1 à 15 atomes de carbone, de préférence un radical méthyle, éthyle, n-propyle, isopropyle, n-butyle, sec-butyle, tert-butyle, n-pentyle, isopentyle, tert-pentyle, n-hexyle, n-heptyle,
. un radical alkyle ayant de 1 à 15 atomes de carbone porteur d'un groupe fonctionnel, de préférence une

fonction OH, CN, $N[R_{10}]_2$, $COOR_{10}$ avec $R_{10}$, identiques ou différents, représentant un atome d'hydrogène, un radical alkyle linéaire ou ramifié ayant de 1 à 6 atomes de carbone : la chaîne alkyle pouvant être interrompue par un atome d'oxygène ou un groupe carbonyle, carboxy ou amino éventuellement substitué et l'on peut citer plus particulièrement un radical de formule $-NHCH_3$ ou $-N(CH_3)_2$, un radical de formule $-CH_2-CH_2-CN$, un radical de formule $-CH_2-CO-(CH_2)_4-COOH$, un radical de formule $COCH(CH_3)_2$, un radical de formule $-(CH_2)_6-COOH$, un radical de formule $-CH_2-COOCH_3$, un radical de formule $-CH_2-COOC_2H_5$, un radical de formule $-CH_2-CH_2-COOCH_3$, un radical de formule $-(CH_2)_6-COOCH_3$, un radical de formule $-(CH_2)_6-COOC_2H_5$, un radical de formule $-(CH_2)_5-COOC_2H_5$, un radical de formule $-CO-CH_3$, un radical de formule $-C(CH_3)_2-CO-CH_3$, un radical de formule $-CH_2.CH_2-CO-(CH_2)_4.CH_3$,

. un radical alcénylène ou alkylidène, linéaire ou ramifié, comportant une ou deux doubles liaisons ayant de 1 à 15 atomes de carbone, de préférence, un radical de formule $-CH_2-CH=CH_2$, un radical de formule $-C(CH_3)=CH_2$, un radical de formule $-CH_2-CH=CH-C_2H_5$, un radical de formule $-CH_2-CH=CH-(CH_2)_2-CH_3$, un radical de formule $-CH=CH-(CH_2)_4-CH_3$, un radical de formule $-CH_2-CH=C(CH_3)_2$, un radical de formule $-CH_2-CH=CCH_3-(CH_2)_2-CH=C(CH_3)_2$,

. un radical $=C(CH_3)_2$ ou $=CH-(CH_2)_2-CH_3$,

. un radical alcénylène ou alkylidène, linéaire ou ramifié, comportant une ou deux doubles liaisons ayant de 1 à 15 atomes de carbone porteur d'un groupe fonctionnel, de préférence une fonction OH, CN, $NH_2$, $COOR_{10}$ dans laquelle $R_{10}$ a la signification donnée précédemment : la chaîne insaturée pouvant être interrompue par un atome d'oxygène, groupe carbonyle ou carboxy et l'on peut mentionner plus spécialement un radical de formule $-CH_2-CH=CH-(CH_2)_3-COOH$, un radical de formule $-CH=CH-C(CH_3)=CH-COOH$, un radical de formule $-CH_2-CH=CH-(CH_2)_3-COOCH_3$, un radical de formule $-CH=CH-C(CH_3)=CH-COOCH_3$, un radical de formule $-CH=CH-CO-CH_3$, un radical de formule $-CH=CH-CO-(CH_2)_4-CH_3$ ou un radical de formule $-CH=CH-CHOH-(CH_2)_4-CH_3$,

. un radical alkoxy linéaire ou ramifié ayant de 1 à 6 atomes de carbone, de préférence un radical méthoxy, éthoxy,

. deux atomes successifs du cycle peuvent être reliés entre eux par un pont époxy ou par un pont tel que les radicaux méthylène dioxy, éthylène dioxy, propylène dioxy,

. un radical de type spiro de formule

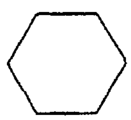 ou 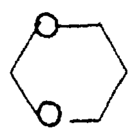 ou 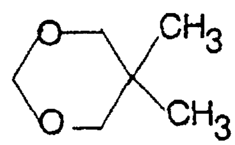

. un radical de formule

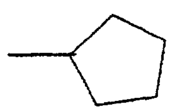 ou 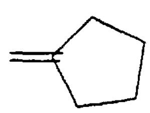 ou 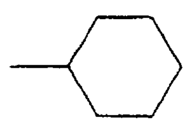 ou 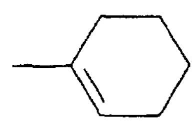

. un radical de formule

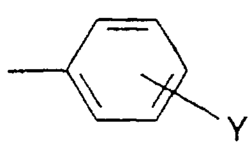 ou 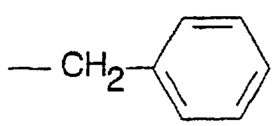 ou 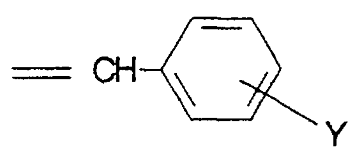

Y représentant un atome d'hydrogène, un atome d'halogène, de préférence de fluor ou de chlore,

. un radical de formule

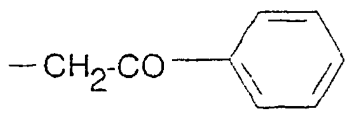

ou

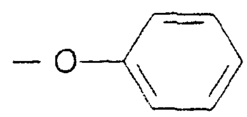

- . un groupe OH,
- . un groupe $COOR_{10}$, $R_{10}$ représentant un atome d'hydrogène ou un radical alkyle ayant de 1 à 6 atomes de carbone, de préférence méthyle, éthyle,
- . un groupe CN,
- . un atome d'halogène, de préférence, de fiuor, de chlore, de brome.

**18.** Procédé selon l'une des revendications 11 et 12, 14 et 15 **caractérisé par le fait que** le composé carbonylé répond à la formule (Ia) dans laquelle A est le reste d'un composé carbocyclique polycyclique, de préférence bicylique comprenant deux carbocycles saturés portant un ou plusieurs substituants tels que :

- . un radical alkyle linéaire ou ramifié ayant de 1 à 6 atomes de carbone, de préférence un radical méthyle, isopropyle,
- . un radical de formule $-CH_2Br$,
- . un radical alkoxy linéaire ou ramifié ayant de 1 à 6 atomes de carbone, de préférence un radical méthoxy,
- . un radical de formule $=CH_2$,
- . un groupe OH,
- . un groupe -COOH,
- . un atome d'halogène, de préférence, de fluor, de chlore, de brome,
- . un groupe $CF_3$

**19.** Procédé selon l'une des revendications 11 et 12, 14 et 15 **caractérisé par le fait que** le composé carbonylé répond à la formule (Ia) dans laquelle A est le reste d'un composé carbocyclique bicylique comprenant deux carbocycles dont l'un est saturé, et l'autre insaturé, portant un ou plusieurs substituants tels que :

- . un radical alkyle linéaire ou ramifié ayant de 1 à 6 atomes de carbone, de préférence un radical méthyle,
- . un radical de formule

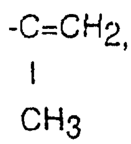

- . un radical de formule $-CH_2-O-CH_3$,
- . un atome d'halogène, de préférence, de chlore.

**20.** Procédé selon l'une des revendications 11 et 12, 14 et 15 **caractérisé par le fait que** le composé carbonylé répond à la formule (Ia) dans laquelle A est le reste d'un composé carbocyclique polycylique, de préférence, bicylique comprenant deux carbocycles insaturés, portant un ou plusieurs radicaux alkyle.

**21.** Procédé selon l'une des revendications 11 et 12, 14 et 15 **caractérisé par le fait que** le composé carbonylé répond à la formule (Ia) dans laquelle A est le reste d'un composé carbocyclique polycylique comprenant au moins un carbocycle aromatique, de préférence, un cycle benzénique, portant un ou plusieurs substituants tels que :

- . un radical alkyle linéaire ou ramifié ayant de 1 à 6 atomes de carbone, de préférence un radical méthyle, tert-butyle,
- . un radical de formule

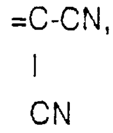

- . un radical alkoxy linéaire ou ramifié ayant de 1 à 6 atomes de carbone, de préférence un radical méthoxy,
- . un radical alkoxy linéaire ou ramifié ayant de 1 à 6 atomes de carbone porteur d'autres groupes fonctionnels tels que, par exemple, un groupe OH et/ou $N[R_{10}]_2$ avec $R_{10}$, identiques ou différents, représentant un atome d'hydrogène, un radical alkyle linéaire ou ramifié ayant de 1 à 6 atomes de carbone, de préférence un radical de formule $-O-CH_2-CHOH-CH_2-NHBu-t$
- . un groupe OH,
- . un groupe acyle ayant de 2 à 6 atomes de carbone, de préférence un radical acétyle ou de formule -CO-tert-butyle,
- . un groupe $-CH_2-COOH$,
- . un groupe $-NH_2$,
- . un atome d'halogène, de préférence, de fluor, de chlore, de brome.

**22.** Procédé selon l'une des revendications 1 à 21 **caractérisé par le fait que** le composé carbonylé mis en oeuvre est choisi parmi les composés suivants :

- benzaldéhyde,
- anisaldéhyde,
- 4-chlorobenzaldéhyde,
- aldéhyde vératrique,
- aldéhyde cinnamique,
- α-naphtylaldéhyde,
- isobornylcyclohexanone,
- isofenchylcyclohexanone,
- isocamphylcyclohexanone,
- bornylcyclohexanone,
- fenchylcyclohexanone,
- camphylcyclohexanone,
- 4-méthylcyclohexanone,
- 4-tert-butylcyclohexanone.

**23.** Procédé selon l'une des revendications 1 à 22 **caractérisé par le fait que** l'alcool mis en oeuvre répond à la formule (III) :

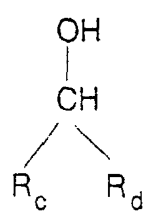

(III)

dans ladite formule (III) :

- $R_c$ et $R_d$ ont la signification donnée précédemment pour $R_a$ et Rb dans la formule (I).

**24.** Procédé selon la revendication 23 **caractérisé par le fait que** l'alcool mis en oeuvre est un alcool aliphatique ou cycloaliphatique, secondaire ou tertiaire.

**25.** Procédé selon l'une des revendications 23 et 24 **caractérisé par le fait que** l'alcool mis en oeuvre est un alcool ayant une faible condensation en carbone, de préférence, moins de 6 atomes de carbone et encore plus préférentiellement moins de 4 atomes de carbone.

**26.** Procédé selon l'une des revendications 23 à 25 **caractérisé par le fait que** l'alcool mis en oeuvre est l'isopropanol, l'isobutanol, le sec-butanol, le tert-butanol, le glycérol.

**27.** Procédé selon l'une des revendications 1 à 26 **caractérisé par le fait que** le catalyseur est une zéolithe naturelle ou synthétique éventuellement désaluminée ou calcinée.

**28.** Procédé selon la revendication 27 **caractérisé par le fait que** la zéolithe est une zéolithe naturelle choisie parmi : la chabazite, la clinoptilolite, l'érionite, la mordénite, la phillipsite, l'offrétite.

**29.** Procédé selon la revendication 27 **caractérisé par le fait que** la zéolithe est une zéolithe synthétique choisie parmi :

- les zèolithes synthétiques à réseau monodimensionnel telles que la zéolithe ZSM-4, la zéolithe L, la zéolithe ZSM-12, la zéolithe ZSM-22, la zéolithe ZSM-23, la zéolithe ZSM-48.
- les zéolithes à réseau bidimensionnel telles que la mordénite, la ferrierite,
- les zéolithes à réseau tridimensionnel telles que la zéolithe β, la zéolithe β au titane, la zéolithe Y, la zéolithe X, la zéolithe ZSM-5, la zéolithe ZSM-11, l'offrétite,
- la zéolithe mésoporeuse de type MCM.

**30.** Procédé selon la revendication 27 **caractérisé par le fait que** la zéolithe est une zéolithe β, une zéolithe β au titane, une zéolithet Y ou de type MCM.

**31.** Procédé selon l'une des revendications 27 à 30 **caractérisé par le fait que** la zéolithe est mise en oeuvre seule ou en mélange avec une matrice minérale choisie, de préférence, parmi les oxydes de métaux, tels que les oxydes d'aluminium, de silicium et/ou de zirconium, ou encore parmi les argiles et plus particulièrement, le kaolin, le talc ou la montmorillonite.

**32.** Procédé selon l'une des revendications 1 à 31 **caractérisé par le fait que** le rapport entre le nombre de moles d'alcool et le nombre de moles de composé carbonylé varie entre 0,1 et 20, et se situe de préférence entre 0,5 et 4,0.

**33.** Procédé selon l'une des revendications 1 à 32 **caractérisé par le fait que** la quantité de catalyseur représente en poids par rapport au composé carbonylé engagée, de 0,01 à 50 %, de préférence, de 1,0 à 20 %.

**34.** Procédé selon l'une des revendications 1 à 33 **caractérisé par le fait que** la température à laquelle est mise en oeuvre la réaction de réduction se situe entre 20°C et 200°C, de préférence entre 40°C et 150°C.

**35.** Procédé selon l'une des revendications 1 à 34 **caractérisé par le fait que** la réaction est conduite sous pression atmosphérique.

**36.** Procédé selon l'une des revendications 1 à 35 **caractérisé par le fait que** le mélange réactionnel traverse le lit catalytique, de préférence, de bas en haut et à sa sortie, est renvoyé dans la zone de mélange des réactifs afin d'être recyclé un nombre de fois suffisant pour obtenir le taux de conversion du substrat souhaité.

**37.** Procédé selon la revendication 36 **caractérisé par le fait que** le taux de conversion est supérieure à 20 %, et de préférence, compris entre 50 et 100 %.

**38.** Procédé selon l'une des revendications 1 à 37 **caractérisé par le fait que** la vitesse linéaire du flux liquide sur le lit catalytique varie entre 0,1 et 10 cm/s, de préférence, entre 0,1 et 5 cm/s.

**39.** Procédé selon l'une des revendications 1 à 38 **caractérisé par le fait que** le temps de séjour du flux de matière sur le lit catalytique varie entre 15 min et 15 h, et de préférence, entre 30 min et 10 h.

**40.** Procédé selon l'une des revendications 1 à 39 **caractérisé par le fait que** l'on obtient en fin de réaction, une phase liquide comprenant l'alcool obtenu qui peut être récupéré de manière classique.

**Patentansprüche**

1. Verfahren zur Reduktion einer Carbonylverbindung zu dem entsprechenden Alkohol durch Reaktion der Carbonylverbindung mit einem Alkohol in Gegenwart eines Katalysators auf Zeolithbasis, **dadurch gekennzeichnet, daß**

   - man die Carbonylverbindung und den Alkohol auf beliebige Art und Weise mischt,
   - man diese Mischung über ein Katalysatorbett, das mindestens einen Zeolith umfaßt, gibt und
   - man die aus dem Katalysatorbett stammende Reaktionsmischung so viele Male einer erneuten Zirkulation über das Katalysatorbett unterwirft, daß es ausreicht, um den gewünschten Umsetzungsgrad in bezug auf das Endprodukt zu erhalten.

2. Verfahren nach Anspruch 1, **dadurch gekennzeichnet, daß** die Carbonylverbindung der allgemeinen Formel (I)

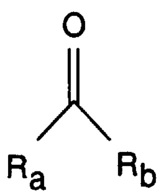

(I)

entspricht, wobei in der Formel (I):

   - $R_a$ und $R_b$, identisch oder verschieden, ein Wasserstoffatom oder einen gegebenenfalls substituierten einwertigen Kohlenwasserstoffrest mit 1 bis 40 Kohlenstoffatomen darstellen, wobei höchstens einer der Reste $R_a$ oder $R_b$ ein Wasserstoffatom ist,
   - $R_a$ und $R_b$ einen Ring, der gegebenenfalls ein weiteres Heteroatom enthält, bilden können.

3. Verfahren nach Anspruch 1 oder 2, **dadurch gekennzeichnet, daß** die Carbonylverbindung der allgemeinen Formel (I) entspricht, in der $R_a$ und $R_b$ einen gegebenenfalls substituierten einwertigen Kohlenwasserstoffrest darstellen, der ein linearer oder verzweigter, gesättigter oder ungesättigter acyclischer aliphatischer Rest oder ein monocyclischer oder polycyclischer, gesättigter, ungesättigter oder aromatischer, heterocyclischer oder carbocyclischer Rest sein kann.

4. Verfahren nach Anspruch 2 oder 3, **dadurch gekennzeichnet, daß** die Carbonylverbindung der allgemeinen Formel (I) entspricht, in der $R_a$ und $R_b$ einen linearen oder verzweigten, gesättigten oder ungesättigten, acyclischen aliphatischen Rest mit vorzugsweise 1 bis 12 Kohlenstoffatomen darstellen, der gesättigt ist oder eine oder mehrere ungesättigte Bindungen in der Kette, im allgemeinen 1 bis 3 ungesättigte Bindungen, umfaßt, bei denen es sich um einfache oder konjugierte Doppelbindungen oder um Dreifachbindungen handeln kann.

5. Verfahren nach einem der Ansprüche 2 bis 4, **dadurch gekennzeichnet, daß** die Carbonylverbindung der allgemeinen Formel (I) entspricht, in der $R_a$ uns $R_b$ einen linearen oder verzweigten Alkyl-, Alkenyl- oder Alkadienylrest mit 1 bis 12 Kohlenstoffatomen darstellen, wobei die Kohlenwasserstoffkette gegebenenfalls

   - durch eine der folgenden Gruppen Z
     -O-; -CO-; COO-; $-NR_1-$; $-CO-NR_1$; -S-; $-SO_2-$; $-NR_1-CO-$;
     unterbrochen sein kann, wobei in diesen Formeln $R_1$ ein Wasserstoffatom oder eine lineare oder verzweigte Alkylgruppe mit 1 bis 6 Kohlenstoffatomen, vorzugsweise einen Methyl- oder Ethylrest, darstellt,
   - und/oder Träger einer der folgenden Substituenten sein kann
     -OH; -COOH; -COOX; $-CO-N(R_1)(R_2)$; $-COOR_1$; -CHO; $-COR_1$; $-NO_2$;
     -X; $-CF_{3,}$

wobei in diesen Formeln X ein Halogenatom, vorzugsweise ein Chloroder Bromatom, darstellt und $R_2$ dieselbe

Bedeutung wie zuvor für $R_1$ angegeben hat.

**6.** Verfahren nach einem der Ansprüche 2 bis 5, **dadurch gekennzeichnet, daß** die Carbonylverbindung der allgemeinen Formel (I) entspricht, in der $R_a$ und $R_b$ einem linearen oder verzweigten, gesättigten oder ungesättigten, acyclischen aliphatischen Rest, der einen ringförmigen Substituenten, vorzugsweise einen Benzolring, trägt, entspricht, wobei der acyclische aliphatische Rest über eine Valenzbindung oder über eine der zuvor in Anspruch 5 genannten Gruppen Z mit dem Ring verbunden sein kann.

**7.** Verfahren nach Anspruch 2 oder 3, **dadurch gekennzeichnet, daß** die Carbonylverbindung der allgemeinen Formel (I) entspricht, in der $R_a$ und $R_b$ darstellen:

- einen monocyclischen, heterocyclischen oder carbocyclischen Rest, der gesättigt ist oder ein oder zwei ungesättigte Bindungen in dem Ring umfaßt, wobei die Zahl der Kohlenstoffatome in dem Ring 3 bis 8 Kohlenstoffatome betragen kann und vorzugsweise 5 oder 6 beträgt,
- einen polycyclischen, vorzugsweise bicyclischen, heterocyclischen oder carbocyclischen Rest, wobei die Zahl der Kohlenstoffatome in dem Ring 3 bis 8 betragen kann und vorzugsweise 5 oder 6 beträgt.

**8.** Verfahren nach Anspruch 2 oder 3, **dadurch gekennzeichnet, daß** die Carbonylverbindung der allgemeinen Formel (I) entspricht, in der $R_a$ und $R_b$ einen aromatischen carbocyclischen Rest, insbesondere einen Benzolrest, darstellen, welche der allgemeinen Formel (II) entspricht

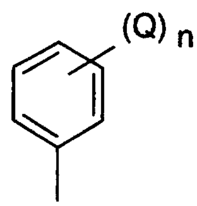

(II)

wobei in der Formel (II)

- n eine ganze Zahl von 0 bis 5, vorzugsweise von 0 bis 3, ist,
- Q einen Rest $R_3$ oder eine der folgenden Gruppen oder Funktionen darstellt:

  · einen linearen oder verzweigten Alkylrest mit 1 bis 6 Kohlenstoffatomen, vorzugsweise 1 bis 4 Kohlenstoffatomen, wie Methyl, Ethyl, Propyl, Isopropyl, Butyl, Isobutyl, sek.-Butyl oder tert.-Butyl,

  · einen linearen oder verzweigten Alkenylrest mit 1 bis 6 Kohlenstoffatomen, vorzugsweise 2 bis 4 Kohlenstoffatomen, wie Vinyl oder Allyl,

  · einen linearen oder verzweigten Alkoxyrest mit 1 bis 6 Kohlenstoffatomen, vorzugsweise 1 bis 4 Kohlenstoffatomen, wie die Reste Methoxy, Ethoxy, Propoxy, Isopropoxy oder Butoxy,

  · eine Acylgruppe mit 2 bis 6 Kohlenstoffatomen,

  · einen Rest der Formel

$$-R_5\text{-}OH$$

$$-R_5\text{-}COOR_7$$

$$-R_5\text{-}CHO$$

$-R_5-NO_2$

$-R_5-CN$

$-R_5-N(R_7)(R_8)$

$-R_5-CO-N(R_7)(R_8)$

$-R_5-SH$

$-R_5-X$

$-R_5-CF_3$

wobei in diesen Formeln $R_5$ eine Valenzbindung oder einen gesättigten oder ungesättigten, linearen oder verzweigten zweiwertigen Kohlenwasserstoffrest mit 1 bis 6 Kohlenstoffatomen, wie Methylen, Ethylen, Propylen, Isopropylen oder Isopropyliden, darstellt; die Reste $R_7$ und $R_8$, identisch oder verschieden, ein Wasserstoffatom oder einen linearen oder verzweigten Alkylrest mit 1 bis 6 Kohlenstoffatomen darstellen; und X ein Halogenatom, vorzugsweise ein Chlor-, Brom- oder Fluoratom, darstellt,

- Q einen Rest $R_4$ oder einen der folgenden komplexeren Reste darstellt

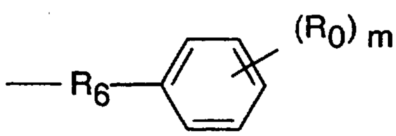

wobei

· m eine ganze Zahl von 0 bis 5, vorzugsweise von 0 bis 3, ist,

· $R_0$ die zuvor für $R_3$ angegebene Bedeutung hat,

· $R_6$ eine Valenzbindung oder eine gesättigte oder ungesättigte, lineare oder verzweigte zweiwertige Kohlenwasserstoffgruppe mit 1 bis 6 Kohlenstoffatomen darstellt, wie Methylen, Ethylen, Propylen, Isopropylen oder Isopropyliden, oder eine der folgenden als Z bezeichneten Gruppen darstellt
-O-; -CO-; COO-; $-NR_7-$; $-CO-NR_7-$; -S-; $-SO_2-$; $-NR_7-CO-$;
wobei in diesen Formeln $R_7$ ein Wasserstoffatom oder eine lineare oder verzweigte Alkylgruppe mit 1 bis 6 Kohlenstoffatomen, vorzugsweise einen Methyl- oder Ethylrest, darstellt.

**9.** Verfahren nach Anspruch 2 oder 3, **dadurch gekennzeichnet, daß** die Carbonylverbindung der allgemeinen Formel (I) entspricht, in der $R_a$ und $R_b$ darstellen:

- einen aromatischen heterocyclischen Rest mit 5 oder 6 Kohlenstoffatomen im Ring, von denen 1 oder 2 Atome Heteroatome sind,

- einen polycyclischen aromatischen heterocyclischen oder carbocyclischen Rest.

**10.** Verfahren nach Anspruch 2 oder 3, **dadurch gekennzeichnet, daß** die Carbonylverbindung der allgemeinen Formel (I) entspricht, in der $R_a$ einen Phenylrest darstellt, der gegebenenfalls eine Alkyl- oder Alkoxygruppe mit 1 bis 4 Kohlenstoffatomen trägt, oder eine Trihalogenmethylgruppe oder ein Halogenatom darstellt und $R_b$ ein Wasserstoffatom ist.

**11.** Verfahren nach Anspruch 1 oder 2, **dadurch gekennzeichnet, daß** die Carbonylverbindung der allgemeinen Formel (Ia) entspricht

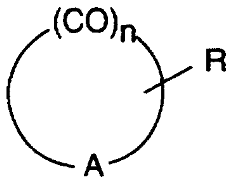

(Ia)

in der:

- A einen Ring darstellt, der insgesamt oder zum Teil ein polycyclisches oder monocyclisches System bildet, das mindestens eine Carbonylgruppe umfaßt,
- R ein Wasserstoffatom oder ein oder mehrere identische oder verschiedene Substituenten darstellt,
- n eine Zahl vorzugsweise von 1 oder 2 ist.

**12.** Verfahren nach Anspruch 1 oder 2, **dadurch gekennzeichnet, daß** die Carbonylverbindung der allgemeinen Formel (Ia), entspricht, in der der gegebenenfalls substituierte Rest A den Rest

- einer gesättigten oder ungesättigten, carbocyclischen monocyclischen Verbindung,
- einer polycyclischen Verbindung, die mindestens zwei gesättigte und/ oder ungesättigte Carbocyclen umfaßt,
- einer polycyclischen Verbindung, die mindestens zwei gesättigte und/ oder ungesättigte Ringe umfaßt, wobei eines oder mehrere der Kohlenstoffatome durch ein Heteroatom ersetzt sein können,
- einer polycyclischen Verbindung, die mindestens zwei Carbocyclen, von denen einer aromatisch ist, umfaßt,

darstellt.

**13.** Verfahren nach Anspruch 1 oder 2, **dadurch gekennzeichnet, daß** die Carbonylverbindung eine carbocyclische monocyclische Verbindung mit einer Kohlenstoffanzahl im Ring zwischen 3 und 20, vorzugsweise 5 oder 6, ist, wobei dieser Carbocyclus gegebenenfalls 1 bis 2 Doppelbindungen umfassen kann.

**14.** Verfahren nach Anspruch 1 oder 2, **dadurch gekennzeichnet, daß** die Carbonylverbindung eine polycyclische Verbindung mit einer Kohlenstoffanzahl im Ring von 3 bis 8 Kohlenstoffatomen, vorzugsweise 5 oder 6 Kohlenstoffatomen, ist.

**15.** Verfahren nach Anspruch 1 oder 2, **dadurch gekennzeichnet, daß** die Carbonylverbindung eine polycyclische Verbindung ist, die mindestens zwei gesättigte und/oder ungesättigte Ringe umfaßt, wobei ein oder zwei der Kohlenstoffatome durch ein Heteroatom, vorzugsweise durch ein Sauerstoff- oder Stickstoffatom, ersetzt sein können.

**16.** Verfahren nach einem der Ansprüche 11 bis 15, **dadurch gekennzeichnet, daß** die Carbonylverbindung der Formel (Ia) entspricht, in welcher A ein oder mehrere Substituenten R trägt, wobei

- R einen Rest $R_9$ oder eine der folgenden Gruppen darstellen kann:

  einen linearen oder verzweigten acyclischen aliphatischen Rest mit 1 bis 20 Kohlenstoffatomen, der gesättigt ist oder eine oder mehrere ungesättigte Bindungen in der Kette, vorzugsweise 1 bis 3 ungesättigte Bindungen, enthält, die vorzugsweise einfache oder konjugierte Doppelbindungen sein können, wobei

die Kohlenwasserstoffkette gegebenenfalls

· unterbrochen sein kann durch eine der folgenden als Z bezeichneten Gruppen
-O-; -CO-; COO-; $-NR_{10}-$; $-CO-NR_{10}-$; -S-; $-SO_2-$;
wobei in diesen Formeln der Rest $R_{10}$ ein Wasserstoffatom oder einen linearen oder verzweigten Alkylrest mit 1 bis 6 Kohlenstoffatomen darstellt,

. und/oder einen der folgenden Substituenten trägt
- OH; -CN; $-N[R_{10}]_2$; $-COOR_{10}$; $-CF_3$ oder -X;
wobei in diesen Formeln die Reste $R_{10}$, identisch oder verschieden, ein Wasserstoffatom oder einen linearen oder verzweigten Alkylrest mit 1 bis 6 Kohlenstoffatomen darstellen und X ein Halogenatom, vorzugsweise Fluor, Chlor oder Brom, darstellt,

· einen Rest vom Typ $=R_{11}$, wobei $R_{11}$ einen Alkylidenrest mit 1 bis 6 Kohlenstoffatomen, einen Rest der Formel $=C(CN)_2$ oder einen Cycloalkylidenrest oder Cycloalkenylidenrest mit 5 oder 6 Kohlenstoffatomen darstellt oder einen gegebenenfalls substituierten, vorzugsweise mit einem Halogenatom X substituierten, Bezylidenrest darstellt,

· einen linearen oder verzweigten Alkoxyrest mit 1 bis 6 Kohlenstoffatomen,

· wobei zwei aufeinanderfolgende Atome des Rings untereinander über eine Epoxybrücke oder über eine Brücke von Alkylendioxytyp mit 1 bis 4 Kohlenstoffatomen, vorzugsweise Methylendioxy, Ethylendioxy oder Propylendioxy, verbunden sein können,

· eine OH-Gruppe,

· eine Gruppe $COOR_{10}$, wobei $R_{10}$ ein Wasserstoffatom oder einen Alkylrest mit 1 bis 6 Kohlenstoffatomen, vorzugsweise Methyl oder Ethyl, darstellt,

· eine CN-Gruppe,

· ein Halogenatom, vorzugsweise Fluor, Chlor oder Brom,

· eine Gruppe $-CF_3$;

- R einen Rest $R_{12}$ oder eine der folgenden komplexeren Gruppe darstellen kann:

· einen gesättigten oder ungesättigten carbocyclischen Rest mit 4 bis 7 Kohlenstoffatomen, vorzugsweise einen Cyclopentyl-, Cyclohexyl-, Cyclopenten-2-yl-, Cyclopenten-3-yl-, Cyclohexen-1-yl-, Cyclohexen-2-yl- oder Cyclohexen-3-yl-Rest,

· einen Rest der Formel

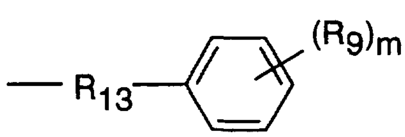

,

in der der Rest $R_{13}$ einer Valenzbindung oder einen gesättigten oder ungesättigten, linearen oder verzweigten zweiwertigen Kohlenwasserstoffrest mit 1 bis 6 Kohlenstoffatomen, wie Methylen, Ethylen, Propylen, Isopropylen oder Isopropyliden, darstellt und der Rest $R_9$ die zuvor angegebene Bedeutung hat und m eine ganze Zahl von 0 bis 4 ist,

· einen Rest $-R_{13}-Z-R_{14}$, wobei Z und $R_{13}$ die zuvor angegebene Bedeutung haben und der Rest $R_{14}$ einen linearen oder verzweigten Alkylrest mit 1 bis 6 Kohlenstoffatomen darstellt, oder einen Rest der Formel

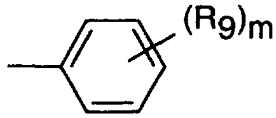

wobei $R_9$ und m die zuvor angegebene Bedeutung haben,

einen Rest vom Spirotyp der Formel

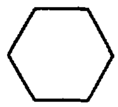 oder 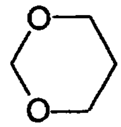 oder 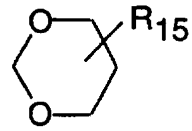

in der $R_{15}$ ein oder mehrere lineare oder verzweigte Alkylreste mit 1 bis 6 Kohlenstoffatomen darstellt.

**17.** Verfahren nach einem der Ansprüche 11 bis 13, **dadurch gekennzeichnet, daß** die Carbonylverbindung der Formel (Ia) entspricht, in der A der Rest einer gesättigten, monocyclischen carbocyclischen Verbindung ist, die eine oder mehrere der folgenden Substituenten trägt:

- einen linearen oder verzweigten Alkylrest mit 1 bis 5 Kohlenstoffatomen, vorzugsweise Methyl, Ethyl, n-Propyl, Isopropyl, n-Butyl, sek.-Butyl, tert.-Butyl, n-Pentyl, Isopentyl, tert.-Pentyl, n-Hexyl oder n-Heptyl,
- einen Alkylrest mit 1 bis 15 Kohlenstoffatomen, der eine funktionelle Gruppe trägt, vorzugsweise eine Funktion OH, CN, $N[R_{10}]_2$, $COOR_{10}$ mit $R_{10}$, identisch oder verschieden, als Wasserstoffatom oder linearer oder verzweigter Alkylrest mit 1 bis 6 Kohlenstoffatomen, wobei die Alkylkette durch ein Sauerstoffatom oder eine Carbonyl-, Carboxy- oder eine gegebenenfalls substituierte Aminogruppe unterbrochen sein kann,

wobei man insbesondere einen Rest der Formel $-NHCH_3$ oder $-N(CH_3)_2$, einen Rest der Formel $-CH_2-CH_2-CN$, einen Rest der Formel $-CH_2-CO-(CH_2)_4-COOH$, einen Rest der Formel $COCH(CH_3)_2$, einen Rest der Formel $-(CH_2)_6-COOH$, einen Rest der Formel $-CH_2-COOCH_3$, einen Rest der Formel $-CH_2-COOC_2H_5$, einen Rest der Formel $-CH_2-CH_2-COOCH_3$, einen Rest der Formel $-(CH_2)_6-COOCH_3$, einen Rest der Formel $-(CH_2)_6-COOC_2H_5$, einen Rest der Formel $-(CH_2)_5-COOC_2H_5$, einen Rest der Formel $-CO-CH_3$, einen Rest der Formel $-C(CH_3)_2-CO-CH_3$ oder einen Rest der Formel $-CH_2-CH_2-CO-(CH_2)_4-CH_3$ nennen kann,

- einen linearen oder verzweigten Alkenylen- oder Alkylidenrest, der ein oder zwei Doppelbindungen mit 1 bis 15 Kohlenstoffatomen, vorzugsweise einen Rest der Formel $-CH_2-CH=CH_2$, einen Rest der Formel $-C(CH_3)=CH_2$, einen Rest der Formel $-CH_2-CH=CH-C_2H_5$, einen Rest der Formel $-CH_2-CH=CH-(CH_2)_2-CH_3$, einen Rest der Formel $-CH=CH-(CH_2)_4-CH_3$, einen Rest der Formel $-CH_2-CH=C(CH_3)_2$ oder einen Rest der Formel $-CH_2-CH=CCH_3-(CH_2)_2-CH=C(CH_3)_2$,
- einen Rest $=C(CH_3)_2$ oder $=CH-(CH_2)_2-CH_3$,
- einen linearen oder verzweigten Alkenylen- oder Alkylidenrest, der ein oder zwei Doppelbindungen mit 1 bis 15 Kohlenstoffatomen umfaßt, die eine funktionelle Gruppe tragen, vorzugsweise eine Funktion OH, CN, $NH_2$, $COOR_{10}$, wobei $R_{10}$ die zuvor angegebene Bedeutung hat, wobei die ungesättigte Kette unterbrochen sein kann durch ein Sauerstoffatom, eine Carbonyl- oder Carboxygruppe und man insbesondere nennen kann einen Rest der Formel $-CH_2-CH=CH-(CH_2)_3-COOH$, eine Rest der Formel $-CH=CH-C(CH_3)=CH-COOH$, einen Rest der Formel $-CH_2-CH=CH-(CH_2)_3-COOCH_3$, einen Rest $-CH=CH-C(CH_3)=CH-COOCH_3$, einen Rest der Formel $-CH=CH-CO-CH_3$, einen Rest der Formel $-CH=CH-CO-(CH_2)_4-CH_3$ oder $-CH=CH-CHOH-(CH_2)_4-CH_3$,
- einen linearen oder verzweigten Alkoxyrest mit 1 bis 6 Kohlenstoffatomen, vorzugsweise Methoxy oder Ethoxy,
- wobei zwei aufeinanderfolgende Atome des Rings untereinander über eine Epoxybrücke oder eine Brücke vom Methylendioxy-, Ethylendioxy-, oder Propylendioxytyp verbunden sein können,

- einen Rest vom Spirotyp der Formel

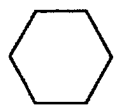 oder 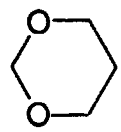 oder 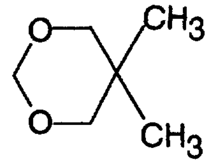

- einen Rest der Formel

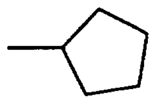 oder 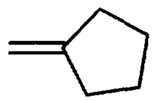 oder 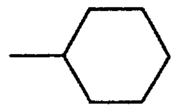 oder 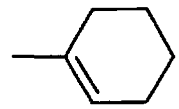

- einen Rest der Formel

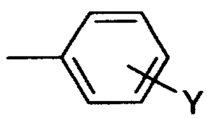 oder 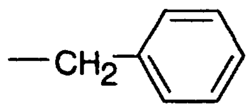 oder 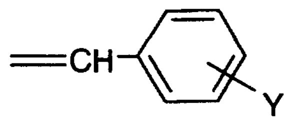

wobei Y ein Wasserstoffatom oder ein Halogenatom, vorzugsweise Fluor oder Chlor, darstellt,

- eine Rest der Formel

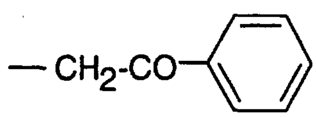 oder 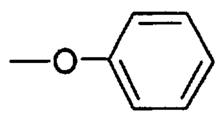

- eine OH-Gruppe,
- eine Gruppe $COOR_{10}$, wobei $R_{10}$ ein Wasserstoffatom oder einen Alkylrest mit 1 bis 6 Kohlenstoffatomen, vorzugsweise Methyl oder Ethyl, darstellt,
- eine CN-Gruppe,
- ein Halogenatom, vorzugsweise Fluor, Chlor oder Brom.

**18.** Verfahren nach einem der Ansprüche 11 und 12 oder 14 und 15, **dadurch gekennzeichnet, daß** die Carbonylverbindung der Formel (Ia) entspricht, in der A der Rest einer polycyclischen, vorzugsweise bicyclischen, carbocyclischen Verbindung ist, der zwei gesättigte Carbocyclen umfaßt, die ein oder mehrere Substituenten tragen können, wie:

- einen linearen oder verzweigten Alkylrest mit 1 bis 6 Kohlenstoffatomen, vorzugsweise Methyl oder Isopropyl,
- einen Rest der Formel $-CH_2Br$,
- einen linearen oder verzweigten Alkoxyrest mit 1 bis 6 Kohlenstoffatomen, vorzugsweise einen Methoxyrest,
- einen Rest der Formel $=CH_2$,

- eine OH-Gruppe,
- eine Gruppe -COOH,
- ein Halogenatom, vorzugsweise Fluor, Chlor oder Brom,
- eine $CF_3$-Gruppe.

**19.** Verfahren nach einem der Ansprüche 11 und 12 oder 14 und 15, **dadurch gekennzeichnet, daß** die Carbonylverbindung der Formel (Ia) entspricht, in der A der Rest einer bicyclischen carbocyclischen Verbindung ist, die zwei Carbocyclen umfaßt, von denen der eine gesättigt und der andere ungesättigt ist und die ein oder mehrere Substituenten tragen, wie:

- einen linearen oder verzweigten Alkylrest mit 1 bis 6 Kohlenstoffatomen, vorzugsweise einen Methylrest,
- einen Rest der Formel

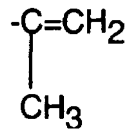

- einen Rest der Formel $-CH_2-O-CH_3$,
- ein Halogenatom, vorzugsweise Chlor.

**20.** Verfahren nach einem der Ansprüche 11 und 12 oder 14 und 15, **dadurch gekennzeichnet, daß** die Carbonylverbindung der Formel (Ia) entspricht, in der A der Rest einer polycyclischen, vorzugsweise bicyclischen, carbocyclischen Verbindung ist, die zwei ungesättigte Carbocyclen umfaßt, die ein oder mehrere Alkylreste tragen.

**21.** Verfahren nach einem der Ansprüche 11 und 12 oder 14 und 15, **dadurch gekennzeichnet, daß** die Carbonylverbindung der Formel (Ia) entspricht, in der A der Rest einer polycyclischen carbocyclischen Verbindung ist, die mindestens einen aromatischen Carbocyclus, vorzugsweise einen Benzolring, umfaßt, der ein oder mehrere Substituenten trägt, wie:

- einen linearen oder verzweigten Alkylrest mit 1 bis 6 Kohlenstoffatomen, vorzugsweise Methyl oder tert.-Butyl,
- einen Rest der Formel

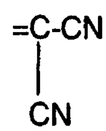

- einen linearen oder verzweigten Alkoxyrest mit 1 bis 6 Kohlenstoffatomen, vorzugsweise Methoxy,
- einen linearen oder verzweigten Alkoxyrest mit 1 bis 6 Kohlenstoffatomen, der weitere funktionelle Gruppen trägt, wie eine OH-Gruppe und/ oder $N[R_{10}]_2$, wobei $R_{10}$, identisch oder verschieden, ein Wasserstoffatom oder einen linearen oder verzweigten Alkylrest mit 1 bis 6 Kohlenstoffatomen, vorzugsweise einen Rest der Formel $-O-CH_2-CHOH-CH_2-NHBu-t$, darstellt,
- eine OH-Gruppe,
- eine Acylgruppe mit 2 bis 6 Kohlenstoffatomen, vorzugsweise einen Acetylrest oder einen Rest der Formel -CO-tert.-Butyl,

- eine Gruppe $-CH_2-COOH$,
- eine Gruppe $-NH_2$,
- ein Halogenatom, vorzugsweise Fluor, Chlor oder Brom.

**22.** Verfahren nach einem der Ansprüche 1 bis 21, **dadurch gekennzeichnet, daß** die eingesetzte Carbonylverbindung ausgewählt ist aus der Gruppe der folgenden Verbindungen:

- Benzaldehyd,
- Anisaldehyd,
- 4-Chlorbenzaldehyd,
- Veratrumaldehyd,
- Zimtaldehyd,
- $\alpha$-Naphtylaldehyd,
- Isobornylcyclohexanon,
- Isofenchylcyclohexanon,
- Isocamphylcyclohexanon,
- Bornylcyclohexanon,
- Fenchylcyclohexanon
- Camphylcyclohexanon
- 4-Methylcyclohexanon
- 4-tert.-Butylcyclohexanon

**23.** Verfahren nach einem der Ansprüche 1 bis 22, **dadurch gekennzeichnet, daß** der eingesetzte Alkohol der Formel (III) entspricht

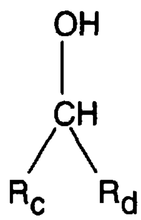

(III)

wobei in der Formel (III)

- $R_c$ und $R_d$ die zuvor für die in der Formel (I) vorkommenden Reste $R_a$ und $R_b$ angegebene Bedeutung haben.

**24.** Verfahren nach Anspruch 23, **dadurch gekennzeichnet, daß** der eingesetzte Alkohol ein sekundärer oder tertiärer, aliphatischer oder cycloaliphatischer Alkohol ist.

**25.** Verfahren nach Anspruch 23 oder 24, **dadurch gekennzeichnet, daß** der eingesetzte Alkohol ein Alkohol mit

geringer Kohlenstoffanzahl, vorzugsweise weniger als 6 Kohlenstoffatomen und besonders bevorzugt weniger als 4 Kohlenstoffatomen, ist.

**26.** Verfahren nach einem der Ansprüche 23 bis 25, **dadurch gekennzeichnet, daß** der eingesetzte Alkohol Isopropanol, Isobutanol, sek.-Butanol, tert.-Butanol oder Glycerol ist.

**27.** Verfahren nach einem der Ansprüche 1 bis 26, **dadurch gekennzeichnet, daß** der Katalysator ein gegebenenfalls desaluminierter oder calcinierter, synthetischer oder natürlicher Zeolith ist.

**28.** Verfahren nach Anspruch 27, **dadurch gekennzeichnet, daß** der Zeolith ein natürlicher Zeolith ist, der ausgewählt ist aus der Gruppe von Chabazit, Clinoptilolit, Erionit, Mordenit, Phillipsit und Offretit.

**29.** Verfahren nach Anspruch 27, **dadurch gekennzeichnet, daß** der Zeolith ein synthetischer Zeolith ist, der ausgewählt ist aus der Gruppe der folgenden Zeolithe:

- eindimensional vernetzte synthetische Zeolithe, wie Zeolith ZSM-4, Zeolith L, Zeolith ZSM-12, Zeolith ZSM-22, Zeolith ZSM-23 oder Zeolith ZSM-48,
- zweidimensional vernetzte Zeolithe, wie Mordenit oder Ferrierit,
- dreidimensional vernetzte Zeolithe, wie β-Zeolith, β-Titanzeolith, Zeolith Y, Zeolith X, Zeolith ZSM-5, Zeolith ZSM-11 oder Offretit,
- mesoporöser Zeolith vom Typ MCM.

**30.** Verfahren nach Anspruch 27, **dadurch gekennzeichnet, daß** der Zeolith ein β-Zeolith, ein β-Titanzeolith oder ein Zeolith Y oder vom Typ MCM ist.

**31.** Verfahren nach einem der Ansprüche 27 bis 30, **dadurch gekennzeichnet, daß** der Zeolith allein oder in Mischung mit einer mineralischen (anorganischen) Matrix eingesetzt wird, die vorzugsweise ausgewählt ist aus Metalloxiden, wie Aluminium-, Silicium- und/oder Zirkoniumoxiden, oder aus Tonen, insbesondere Kaolin, Talk oder Montmorillonit.

**32.** Verfahren nach einem der Ansprüche 1 bis 31, **dadurch gekennzeichnet, daß** das Verhältnis zwischen der Molzahl an Alkohol und der Molzahl an Carbonylverbindung zwischen 0,1 und 20 variiert und vorzugsweise zwischen 0,5 und 4,0 liegt.

**33.** Verfahren nach einem der Ansprüche 1 bis 32, **dadurch gekennzeichnet, daß** die Menge an Katalysator, gewichtsbezogen auf die eingesetzte Carbonylverbindung, 0,01 bis 50 %, vorzugsweise 1,0 bis 20 %, beträgt.

**34.** Verfahren nach einem der Ansprüche 1 bis 33, **dadurch gekennzeichnet, daß** die Temperatur, bei der die Reduktionsreaktion durchgeführt wird, zwischen 20 °C und 200 °C, vorzugsweise zwischen 40 °C und 150°C, liegt.

**35.** Verfahren nach einem der Ansprüche 1 bis 34, **dadurch gekennzeichnet, daß** die Reaktion unter Atmosphärendruck durchgeführt wird.

**36.** Verfahren nach einem der Ansprüche 1 bis 35, **dadurch gekennzeichnet, daß** die Reaktionsmischung das Katalysatorbett durchquert, vorzugsweise von unten nach oben, und am Ausgang in die Mischungszone der Reagenzien zurückgefördert wird, um so viele Male rezykliert zu werden, daß es ausreicht, um den gewünschten Umsetzungsgrad in bezug auf das Endprodukt zu erhalten.

**37.** Verfahren nach Anspruch 36, **dadurch gekennzeichnet, daß** der Umsetzungsgrad größer als 20 % ist und vorzugsweise zwischen 50 und 100 % liegt.

**38.** Verfahren nach einem der Ansprüche 1 bis 37, **dadurch gekennzeichnet, daß** die lineare Geschwindigkeit des Flüssigkeitsdurchflusses oder -durchsatzes über das Katalysatorbett zwischen 0,1 und 10 cm/s, vorzugsweise zwischen 0,1 und 5 cm/s, variiert.

**39.** Verfahren nach einem der Ansprüche 1 bis 38, **dadurch gekennzeichnet, daß** die Verweildauer des Substanzflusses oder -durchsatzes über das Katalysatorbett zwischen 15 Minuten und 15 Stunden, vorzugsweise zwischen 30 Minuten und 10 Stunden, variiert.

**40.** Verfahren nach einem der Ansprüche 1 bis 39, **dadurch gekennzeichnet, daß** man am Ende der Reaktion eine flüssige Phase erhält, die den erhaltenen Alkohol enthält, der auf herkömmliche Art und Weise erhalten werden kann.

## Claims

**1.** A process for reduction of a carbonyl compound to the corresponding alcohol, by reaction of the said carbonyl compound with an alcohol in the presence of a zeolite catalyst,
**characterised in that** it consists of:

- mixing the carbonyl compound and the alcohol,
- passing the said mixture over a catalyst bed containing at least one zeolite,
- subjecting the reaction mixture leaving the catalyst bed to recirculation through the catalyst bed, for a number of times sufficient to obtain the desired degree of conversion of the substrate.

**2.** A process according to claim 1, **characterised in that** the carbonyl compound corresponds to general formula (I):

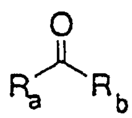 (I)

in the said formula (I):

- $R_a$ and $R_b$, which may be identical or different, represent a hydrogen atom or a monovalent hydrocarbon radical, optionally substituted, possessing from 1 to 40 carbon atoms; at most one of the $R_a$ and $R_b$ radicals is a hydrogen atom,
- $R_a$ and $R_b$ can form a ring, optionally containing another heteroatom.

**3.** A process according to one of claims 1 and 2, **characterised in that** the carbonyl compound corresponds to general formula (I) in which $R_a$ and $R_b$ represent a monovalent hydrocarbon radical, substituted or not, which can be a saturated or unsaturated, linear or branched acyclic aliphatic radical; a monocyclic or polycyclic, saturated, unsaturated or aromatic, heterocyclic or carbocyclic radical.

**4.** A process according to one of claims 2 and 3, **characterised in that** the carbonyl compound corresponds to general formula (I) in which $R_a$ and $R_b$ represent a saturated or unsaturated, linear or branched acyclic aliphatic radical preferably possessing from 1 to 12 carbon atoms, saturated or containing one or more unsaturations on the chain, generally 1 to 3 unsaturations which can be simple or conjugated double bonds or triple bonds.

**5.** A process according to one of claims 2 to 4, **characterised in that** the carbonyl compound corresponds to general formula (I) in which $R_a$ and $R_b$ represent a linear or branched alkyl, alkenyl, alkadienyl radical preferably possessing from 1 to 12 carbon atoms: the hydrocarbon chain can optionally:

- be interrupted by one of the following groups Z:
  $-O-$; $-CO-$; $COO-$; $-NR_1-$; $-CO-NR_1-$; $-S-$; $-SO_2-$; $-NR_1-CO$;
  in the said formulae $R_1$ represents a hydrogen atom, or a linear or branched alkyl group possessing from 1 to 6 carbon atoms, preferably a methyl or ethyl radical,
- and/or it can carry one of the following substituents:
  $OH$; $-COOH$; $-COOX$; $-CO-N(R_1)(R_2)$; $-COOR_1$; $-CHO$; $-COR_1$; $-NO_2$; $-X$; $-CF_3$.
  in these formulae, X represents a halogen atom, preferably a chlorine or bromine atom and $R_2$ has the same meaning as $R_1$ given previously.

6. A process according to one of claims 2 to 5, **characterised in that** the carbonyl compound corresponds to general formula (I) in which $R_a$ and $R_b$ represent a saturated or unsaturated, linear or branched, acyclic aliphatic radical carrying a cyclic substituent, preferably a benzene ring: the acyclic aliphatic radical optionally being joined to the ring by a covalent bond or by one of the groups Z previously mentioned in claim 5.

7. A process according to one of claims 2 and 3, **characterised in that** the carbonyl compound corresponds to general formula (I) in which $R_a$ and $R_b$ represent:

- a monocyclic, heterocyclic or carbocyclic radical, saturated or containing 1 or 2 unsaturations in the ring; the number of carbon atoms in the ring can vary from 3 to 8 carbon atoms and is preferably equal to 5 or 6,
- a polycyclic, heterocyclic or carbocyclic radical, preferably bicyclic; the number of carbon atoms in the ring can vary from 3 to 8 carbon atoms and is preferably equal to 5 or 6.

8. A process according to one of claims 2 and 3, **characterised in that** the carbonyl compound corresponds to general formula (I) in which $R_a$ and $R_b$ represent an aromatic carbocyclic radical, and in particular a benzene radical corresponding to general formula (II):

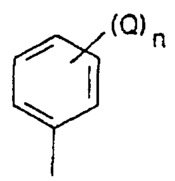

(II)

in the said formula (II):

- n is an integer from 0 to 5, preferably from 0 to 3,
- Q represents $R_3$, one of the following groups or functions:

- a linear or branched alkyl radical, possessing from 1 to 6 carbon atoms, preferably from 1 to 4 carbon atoms, such as methyl, ethyl, propyl, isopropyl, butyl, isobutyl, sec-butyl, tert-butyl,
- a linear or branched alkenyl radical possessing from 2 to 6 carbon atoms, preferably from 2 to 4 carbon atoms, such as vinyl, allyl,
- a linear or branched alkoxy radical possessing from 1 to 6 carbon atoms, preferably from 1 to 4 carbon atoms such as the methoxy, ethoxy, propoxy, isopropoxy or butoxy radicals,
- an acyl group possessing from 2 to 6 carbon atoms,
- a radical with the formula:

$$-R_5\text{-}OH$$

$$-R_5\text{-}COOR_7$$

$$-R_5\text{-}CHO$$

$$-R_5\text{-}NO_2$$

$$-R_5\text{-}CN$$

$$-R_5\text{-}N(R_7)(R_8)$$

- $R_5$-CO-N($R_7$)($R_8$)

- $R_5$-SH

- $R_5$-X

- $R_5$-$CF_3$

in the said formulae, $R_5$ represents a covalent bond or a linear or branched, saturated or unsaturated divalent hydrocarbon radical, possessing from 1 to 6 carbon atoms, for example methylene, ethylene, propylene, isopropylene, isopropylidene; the $R_7$ and $R_8$ radicals, which may be identical or different, represent a hydrogen atom or a linear or branched alkyl radical possessing from 1 to 6 carbon atoms; X denotes a halogen atom, preferably a chlorine, bromine or fluorine atom.

- Q represents $R_4$, one of the following more complex radicals:

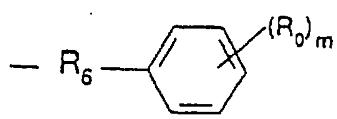

in which:

- m is an integer from 0 to 5, preferably from 0 to 3,
- $R_0$ has the meaning given previously for $R_3$,
- $R_6$ represents a covalent bond; a linear or branched, saturated or unsaturated divalent hydrocarbon group possessing from 1 to 6 carbon atoms, for example methylene, ethylene, propylene, isopropylene, isopropylidene or one of the following groups designated Z:
  -O-; -CO-; COO-; -$NR_7$-; -CO-$NR_7$-; -S-; -$SO_2$-; -$NR_7$-CO-;
  in the said formulae $R_7$ represents a hydrogen atom, a linear or branched alkyl group possessing from 1 to 6 carbon atoms, preferably a methyl or ethyl radical.

**9.** A process according to one of claims 2 and 3, **characterised in that** the carbonyl compound corresponds to general formula (I) in which $R_a$ and $R_b$ represent:

- an aromatic heterocyclic radical containing 5 or 6 atoms in the ring of which 1 or 2 are heteroatoms,
- a polycyclic aromatic heterocyclic or carbocyclic radical.

**10.** A process according to one of claims 2 and 3, **characterised in that** the carbonyl compound corresponds to general formula (I) in which $R_a$ represents a phenyl radical which optionally carries an alkyl or alkoxy group possessing from 1 to 4 carbon atoms, a trihalogenomethyl group or a halogen atom and $R_b$ represents a hydrogen atom.

**11.** A process according to one of claims 1 and 2, **characterised in that** the carbonyl compound corresponds to general formula (Ia):

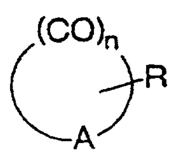

(Ia)

in which:

- A denotes the remainder of a ring forming all or part of a monocyclic or polycyclic system containing at least one carbonyl group,
- R represents a hydrogen atom or one or more substituents, which may be identical or different,
- n is a number preferably equal to 1 or 2.

**12.** A process according to one of claims 1 and 2, **characterised in that** the carbonyl compound corresponds to formula (Ia) in which the remainder A, optionally substituted, represents the remainder:

- of a saturated or unsaturated carbocyclic or monocyclic compound,
- of a polycyclic compound containing at least two carbocycles, saturated and/or unsaturated,
- of a polycyclic compound containing at least two saturated and/or unsaturated rings: one or more of the carbon atoms being replaceable by a heteroatom,
- of a polycyclic compound containing at least two carbocycles, one of which is aromatic.

**13.** A process according to one of claims 1 and 2, **characterised in that** the carbonyl compound is a carbocyclic monocyclic compound with a number of carbon atoms in the ring varying between 3 and 20 carbon atoms, preferably equal to 5 or 6 carbon atoms; the said carbocycle optionally containing 1 to 2 double bonds.

**14.** A process according to one of claims 1 and 2, **characterised in that** the carbonyl compound is a polycyclic compound with a number of carbon atoms in each ring varying between 3 and 8 carbon atoms, preferably equal to 5 or 6 carbon atoms.

**15.** A process according to one of claims 1 and 2, **characterised in that** the carbonyl compound is a polycyclic compound containing at least two saturated and/or unsaturated rings: one or two of the carbon atoms being replaceable by a heteroatom, preferably an oxygen or nitrogen atom.

**16.** A process according to one of claims 11 to 15, **characterised in that** the carbonyl compound corresponds to formula (Ia) in which the remainder A carries one or more substituents R such that:

- R can represent $R_9$, one of the following groups:

- a linear or branched, acyclic aliphatic radical, possessing from 1 to 20 carbon atoms, saturated or containing one or more on the chain, preferably 1 to 3 unsaturations which are preferably simple or conjugated double bonds; the hydrocarbon chain optionally being:

- interrupted by one of the following groups designated Z:
  $-O-$; $-CO-$; $COO-$; $-NR_{10}-$; $-CO-NR_{10}-$; $-S-$; $-SO_2-$;
  in the said formulae $R_{10}$ represents a hydrogen atom, a linear or branched alkyl radical possessing from 1 to 6 carbon atoms,
- and/or carrying one of the following substituents:

- OH; -CN; $-N[R_{10}]_2$; $-COOR_{10}$; $-CF_3$ or -X; in the said formulae, the $R_{10}$ radicals, which may be identical or different, represent a hydrogen atom, a linear or branched alkyl radical possessing from 1 to 6 carbon atoms and X represents a halogen atom, preferably fluorine, chlorine or bromine,
- a radical of the type $=R_{11}$, in which $R_{11}$ represents an alkylidene radical possessing from 1 to 6 carbon atoms, a radical with the formula $=C(CN)_2$ or a cycloalkylidene or cycloalkenylidene radical possessing 5 or 6 carbon atoms or a benzylidene radical, optionally substituted, preferably by a halogen atom X,
- a linear or branched alkoxy radical possessing from 1 to 6 carbon atoms,
- two successive atoms of the ring can be joined together by an epoxy bridge or by a bridge of the alkylene dioxy type possessing from 1 to 4 carbon atoms, preferably the methylene dioxy, ethylene dioxy, propylene dioxy radicals,
- an OH group
- a $COOR_{10}$ group, $R_{10}$ representing a hydrogen atom or an alkyl radical possessing from 1 to 6 carbon atoms, preferably methyl, ethyl,
- a CN group,
- a halogen atom, preferably fluorine, chlorine or bromine,

- a-$CF_3$ group.
- R can represent $R_{12}$, one of the following more complex groups:

- a saturated or unsaturated carbocyclic radical possessing from 4 to 7 carbon atoms, preferably a cyclopentyl, cyclohexyl, cyclopenten-2-yl, cyclopenten-3-yl, cyclohexen-1-yl, cyclohexen-2-yl or cyclohexen-3-yl radical,
- a radical with the formula

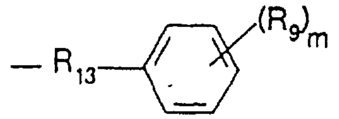

  in which $R_{13}$ represents a covalent bond or a linear or branched, saturated or unsaturated divalent hydrocarbon radical possessing from 1 to 6 carbon atoms, for example methylene, ethylene, propylene, isopropylene, isopropylidene and $R_9$ has the meaning given previously and m is an integer from 0 to 4,
- a $-R_{13}-Z-R_{14}$ radical in which Z and $R_{13}$ have the meaning given previously, $R_{14}$ represents a linear or branched alkyl radical possessing from 1 to 6 carbon atoms or a radical with the formula

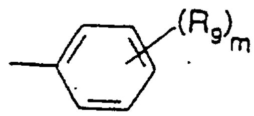

- in which $R_9$ and m have the meaning given previously,
- a radical of the spiro type with the formula:

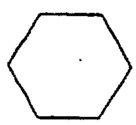 or 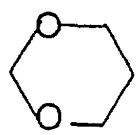 or 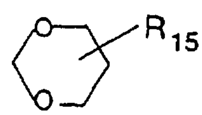

  in which $R_{15}$ represents one or more linear or branched alkyl radicals possessing from 1 to 6 carbon atoms.

**17.** A process according to one of claims 11 to 13, **characterised in that** the carbonyl compound corresponds to formula (Ia) in which A is the remainder of a saturated monocyclic carbocyclic compound carrying one or more substituents such as:

- a linear or branched alkyl radical possessing from 1 to 15 carbon atoms, preferably a methyl, ethyl, n-propyl, isopropyl, n-butyl, sec-butyl, tert-butyl, n-pentyl, isopentyl, tert-pentyl, n-hexyl, n-heptyl radical,
- an alkyl radical possessing from 1 to 15 carbon atoms, carrying a functional group preferably an OH, CN, $N[R_{10}]_2$, $COOR_{10}$ function with $R_{10}$, which may be identical or different, representing a hydrogen atom, a linear or branched alkyl radical possessing from 1 to 6 carbon atoms: the alkyl chain can be interrupted by an oxygen atom or a carbonyl, carboxy or amino group, optionally substituted, and there can be mentioned more particularly a radical with the formula $-NHCH_3$ or $-N(CH_3)_2$, a radical with the formula $-CH_2-CH_2-CN$, a radical with the formula $-CH_2-CO-(CH_2)_4-COOH$, a radical with the formula $COCH(CH_3)_2$, a radical with the formula $-(CH_2)_6-COOH$, a radical with the formula $-CH_2-COOCH_3$, a radical with the formula $-CH_2-COOC_2H_5$, a radical with the formula $-CH_2-CH_2-COOCH_3$, a radical with the formula $-(CH_2)_6-COOCH_3$, a radical with the formula $-(CH_2)_6-COOC_2H_5$, a radical with the formula $-(CH_2)_5-COOC_2H_5$, a radical with the formula $-CO-CH_3$, a radical with the formula $-C(CH_3)_2-CO-CH_3$, a radical with the formula $-CH_2-CH_2-CO-(CH_2)_4-CH_3$,
- a linear or branched alkenylene or alkylidene radical, containing one or two double bonds possessing from 1 to 15 carbon atoms, preferably a radical with the formula $-CH_2-CH=CH_2$, a radical with the formula $-C(CH_3)=CH_2$, a radical with the formula $-CH_2-CH=CH-C_2H_5$, a radical with the formula $-CH_2-CH=CH-(CH_2)_2-CH_3$, a radical with the formula $-CH=CH-(CH_2)_4-CH_3$, a radical with the formula $-CH_2-CH=C(CH_3)_2$, a radical with the formula $-CH_2-CH=CCH_3-(CH_2)_2-CH=C(CH_3)_2$,

- a $=C(CH_3)_2$ or $=CH-(CH_2)_2-CH_3$ radical,
- a linear or branched alkenylene or alkylidene radical, containing one or two double bonds possessing from 1 to 15 carbon atoms, carrying a functional group, preferably an OH, CN, $NH_2$, $COOR_{10}$ function in which $R_{10}$ has the meaning given previously: the unsaturated chain can be interrupted by an oxygen atom, a carbonyl or carboxy group and there can be mentioned more especially a radical with the formula $-CH_2-CH=CH-(CH_2)_3-COOH$, a radical with the formula $-CH=CH-C(CH_3)=CH-COOH$, a radical with the formula $-CH_2-CH=CH-(CH_2)_3-COOCH_3$, a radical with the formula $-CH=CH-C(CH_3)=CH-COOCH_3$, a radical with the formula $-CH=CH-CO-CH_3$, a radical with the formula $-CH=CH-CO-(CH_2)_4-CH_3$ or a radical with the formula $-CH=CH-CHOH-(CH_2)_4-CH_3$,
- a linear or branched alkoxy radical possessing from 1 to 6 carbon atoms, preferably a methoxy or ethoxy radical,
- two successive atoms of the ring can be joined together by an epoxy bridge or by a bridge such as the methylene dioxy, ethylene dioxy, propylene dioxy radicals,
- a radical of the spiro type with the formula

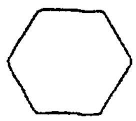
or
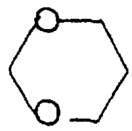
or
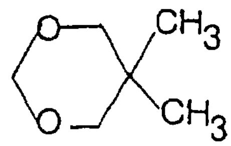

- a radical with the formula

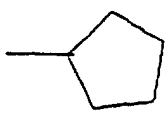
or
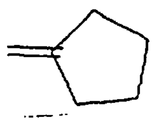
or
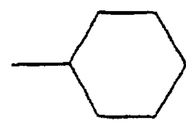
or
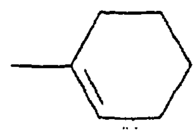

- a radical with the formula

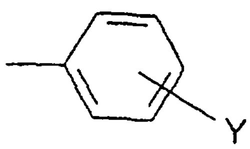
or
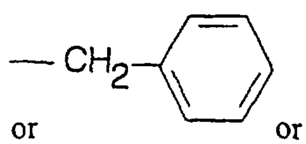
or
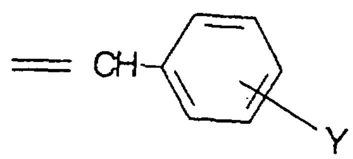

Y representing a hydrogen atom, a halogen atom, preferably fluorine or chlorine,

- a radical with the formula

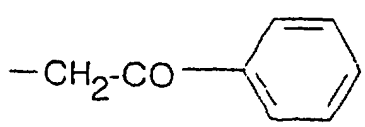
or
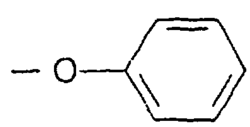

- an OH group,
- a $COOR_{10}$ group, $R_{10}$ representing a hydrogen atom or an alkyl radical possessing from 1 to 6 carbon atoms, preferably methyl, ethyl,
- a CN group,
- a halogen atom, preferably fluorine, chlorine or bromine.

**18.** A process according to one of claims 11 and 12, 14 and 15, **characterised in that** the carbonyl compound corresponds to formula (Ia) in which A is the remainder of a polycyclic carbocyclic compound, preferably bicyclic, containing two saturated carbocycles carrying one or more substituents such as:

- a linear or branched alkyl radical possessing from 1 to 6 carbon atoms, preferably a methyl or isopropyl radical,
- a radical with the formula $-CH_2Br$,
- a linear or branched alkoxy radical possessing from 1 to 6 carbon atoms, preferably a methoxy radical,
- a radical with the formula $=CH_2$,
- an OH group,
- a -COOH group,
- a halogen atom, preferably fluorine, chlorine or bromine,
- a $CF_3$ group

**19.** A process according to one of claims 11 and 12, 14 and 15, **characterised in that** the carbonyl compound corresponds to formula (Ia) in which A is the remainder of a bicyclic carbocyclic compound containing two carbocycles, one of which is saturated and the other is unsaturated, carrying one or more substituents such as:

- a linear or branched alkyl radical possessing from 1 to 6 carbon atoms, preferably a methyl radical,
- a radical with the formula

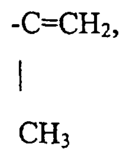

- a radical with the formula $-CH_2-O-CH_3$,
- a halogen atom, preferably chlorine.

**20.** A process according to one of claims 11 and 12, 14 and 15, **characterised in that** the carbonyl compound corresponds to formula (Ia) in which A is the remainder of a polycyclic, preferably bicyclic, carbocyclic compound containing two unsaturated carbocycles, carrying one or more alkyl radicals.

**21.** A process according to one of claims 11 and 12, 14 and 15, **characterised in that** the carbonyl compound corresponds to formula (Ia) in which A is the remainder of a polycyclic carbocyclic compound containing at least one aromatic carbocycle, preferably a benzene ring, carrying one or more substituents such as:

- a linear or branched alkyl radical possessing from 1 to 6 carbon atoms, preferably a methyl or tert-butyl radical,
- a radical with the formula

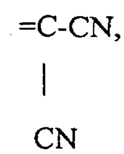

- a linear or branched alkoxy radical possessing from 1 to 6 carbon atoms, preferably a methoxy radical,
- a linear or branched alkoxy radical possessing from I to 6 carbon atoms, carrying other functional groups such as an OH and/or $N[R_{10}]_2$ group with $R_{10}$, which may be identical or different, representing a hydrogen atom, a linear or branched alkyl radical possessing from 1 to 6 carbon atoms, preferably a radical with the formula $-O-CH_2-CHOH-CH_2-NHBu-t$
- an OH group,
- an acyl group possessing from 2 to 6 carbon atoms, preferably an acetyl radical or a radical with the formula -CO-tert-butyl,
- a $-CH_2-COOH$ group,
- an $-NH_2$ group,
- a halogen atom, preferably fluorine, chlorine or bromine.

**22.** A process according to one of claims 1 to 21, **characterised in that** the carbonyl compound employed is chosen from the following compounds:

- benzaldehyde,
- anisaldehyde,
- 4-chlorobenzaldehyde,
- veratric aldehyde,
- cinnamic aldehyde,
- $\alpha$-naphthylaldehyde,
- isobornylcyclohexanone,
- isofenchylcyclohexanone
- isocamphylcyclohexanone,
- bornylcyclohexanone,
- fenchylcyclohexanone,
- camphylcyclohexanone
- 4-methylcyclohexanone,
- 4-tert-butylcyclohexanone.

**23.** A process according to one of claims 1 to 22, **characterised in that** the alcohol employed corresponds to formula (III):

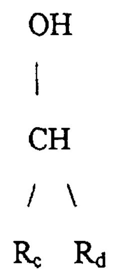

(III)

in the said formula (III):

- $R_c$ and $R_d$ have the meaning given previously for $R_a$ and $R_b$ in formula (I).

**24.** A process according to claim 23, **characterised in that** the alcohol employed is a secondary or tertiary, aliphatic or cycloaliphatic alcohol.

**25.** A process according to one of claims 23 and 24, **characterised in that** the alcohol employed is an alcohol that has a slight condensation to carbon, preferably less than 6 carbon atoms and even more preferably, less than 4 carbon atoms.

**26.** A process according to one of claims 23 to 25, **characterised in that** the alcohol employed is isopropanol, isobutanol, sec-butanol, tert-butanol or glycerol.

**27.** A process according to one of claims 1 to 26, **characterised in that** the catalyst is a natural or synthetic zeolite, optionally dealuminated or calcined.

**28.** A process according to claim 27, **characterised in that** the zeolite is a natural zeolite chosen from: chabazite, clinoptilolite, erionite, mordenite, phillipsite, offretite.

**29.** A process according to claim 27, **characterised in that** the zeolite is a synthetic zeolite chosen from:

- the synthetic zeolites with a one-dimensional network such as zeolite ZSM-4, zeolite L, zeolite ZSM-12, zeolite ZSM-22, zeolite ZSM-23, zeolite ZSM-48.
- the zeolites with a two-dimensional network such as mordenite, ferrierite,
- the zeolites with a three-dimensional network such as zeolite $\beta$, titanium zeolite $\beta$, zeolite Y, zeolite X, zeolite

ZSM-5, zeolite ZSM-11, offretite.
- the mesoporous zeolite of the MCM type.

**30.** A process according to claim 27, **characterised in that** the zeolite is a zeolite β, a titanium zeolite β, a zeolite Y or of the MCM type.

**31.** A process according to one of claims 27 to 30, **characterised in that** the zeolite is employed alone or mixed with a mineral matrix chosen, preferably, from the oxides of metals, such as the oxides of aluminium, silicon and/or zirconium, or alternatively from clays and more particularly kaolin, talc or montmorillonite.

**32.** A process according to one of claims 1 to 31, **characterised in that** the ratio between the number of moles of alcohol and the number of moles of carbonyl compound varies between 0.1 and 20, and is preferably between 0.5 and 4.0.

**33.** A process according to one of claims 1 to 32, **characterised in that** the amount of catalyst represents by weight, relative to the carbonyl compound employed, from 0.01 to 50%, preferably from 1.0 to 20%.

**34.** A process according to one of claims 1 to 33, **characterised in that** the temperature at which the reduction reaction is carried out is between 20°C and 200°C, preferably between 40°C and 150°C.

**35.** A process according to one of claims 1 to 34, **characterised in that** the reaction is carried out at atmospheric pressure.

**36.** A process according to one of claims 1 to 35, **characterised in that** the reaction mixture passes through the catalyst bed preferably from bottom to top and on leaving, is sent to the reactant mixing zone to be recycled for a number of times that is sufficient to obtain the desired degree of conversion of the substrate.

**37.** A process according to claim 36, **characterised in that** the degree of conversion is greater than 20%, and preferably is between 50 and 100%.

**38.** A process according to one of claims 1 to 37, **characterised in that** the linear speed of liquid flow through the catalyst bed varies between 0.1 and 10 cm/s, and preferably between 0.1 and 5 cm/s.

**39.** A process according to one of claims 1 to 38, **characterised in that** the dwell time of the material flow through the catalyst bed varies between 15 min and 15 h, and preferably between 30 min and 10 h.

**40.** A process according to one of claims 1 to 39, **characterised in that** we obtain, at the end of reaction, a liquid phase containing the alcohol obtained, which can be recovered in the conventional manner.

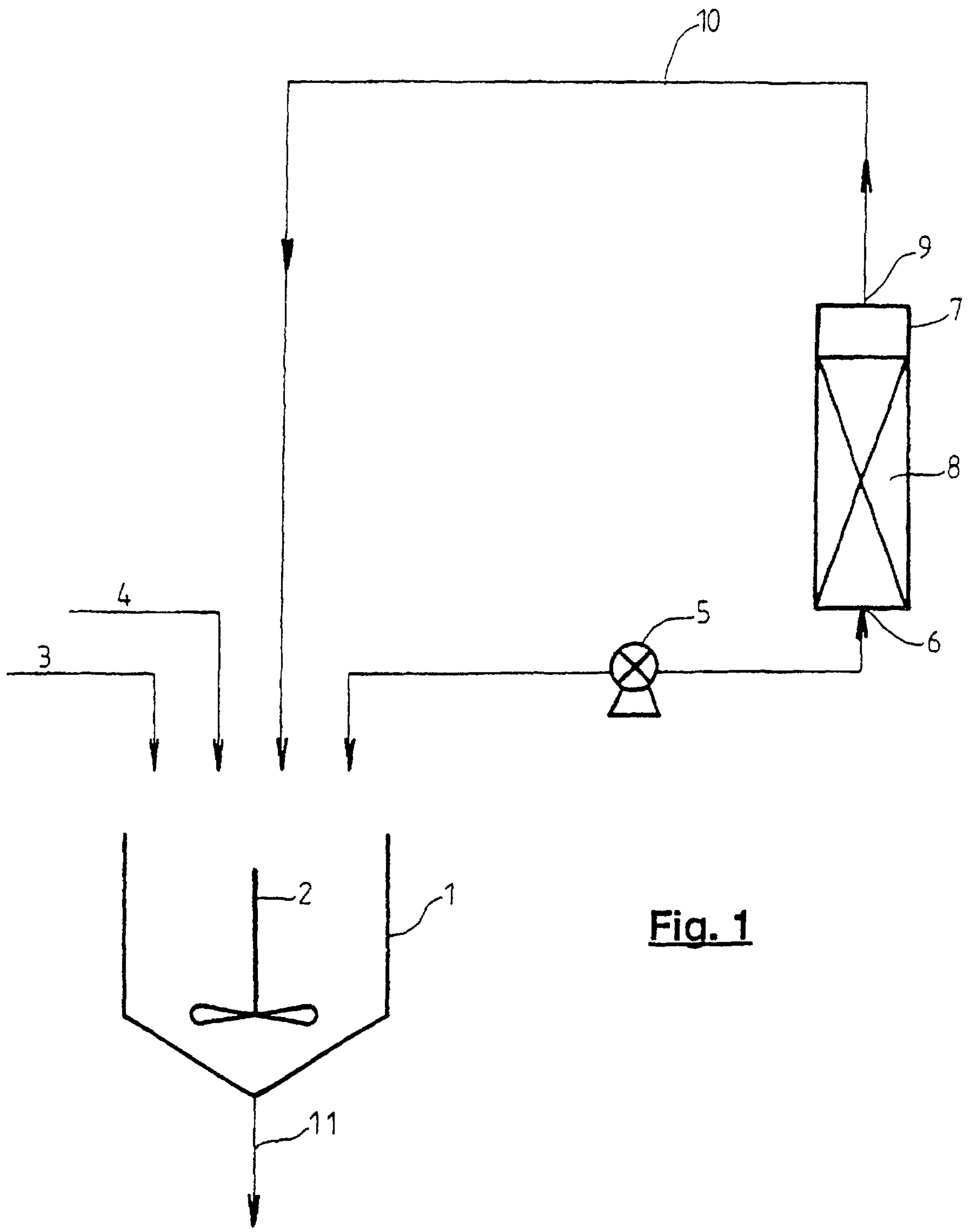


Fig. 1